# EUROPEAN PATENT SPECIFICATION

(11) **EP 2 985 279 B1**
(45) Date of publication and mention of the grant of the patent: **02.08.2017**
(21) Application number: 14180847.7
(22) Date of filing: 13.08.2014
(51) Int. Cl.: C07D 277/26, A61K 31/426, A61P 25/00

(54) **Heterocyclic compounds with working memory enhancing activity**
Heterocyclische Verbindungen mit Aktivität zur Arbeitsspeicherverbesserung
Composés hétérocycliques ayant une activité d'amélioration de la mémoire de travail

(43) Date of publication of application: 17.02.2016
(73) Proprietor: Red Bull GmbH, 5330 Fuschl am See (AT)
(72) Inventor: LEBAN, Johann, 1190 Wien (AT); LUBEC, Gert, 1220 Wien (AT)
(74) Representative: Metten, Karl-Heinz

(56) References cited:
- US-A1- 2002 183 334
- US-A1- 2005 234 040
- , 26 May 2016 (2016-05-26), Retrieved from the Internet: URL:https://en.wikipedia.org/wiki/Desmethy lclozapine [retrieved on 2017-02-28]
- , 4 May 2016 (2016-05-04), Retrieved from the Internet: URL:https://en.wikipedia.org/wiki/Desmethy l [retrieved on 2017-02-28]

## Description

The present invention relates to novel compounds that can be used to improve cognitive functions and in particular short term memory and/or working memory in human individuals, in particular in aging individuals. In particular, the present invention refers to new compounds which can improve working memory performance.

Working memory is the system that actively holds multiple pieces of transitory information in the mind, where they can be manipulated in a way that makes them useful for goal directed behaviour. This involves execution of verbal and nonverbal tasks, such as reasoning and comprehension and makes them available for further information-processing (Becker JT et al. 1999 Brain Cogn 41 (1): 1-8). That is, it is considered that the working memory includes subsystems that store and manipulate visual images or verbal information, resp., and also a central executive that coordinates these subsystems. It has been proposed to include visual representation of the possible moves, and awareness of the flow of information into and out of memory, all stored for a limited amount of time (Schacter, Daniel (2009, 2011), Psychology Second Edition, United States of America, Worth Publishers, p. 227). The concept of a working memory is generally based on the Baddeley's mode as proposed in 1974 and supplemented in 2000 (Baddeley, Alan D.; Hitch, Graham (1974). "Working Memory"., in Gordon H. Bower: "The psychology of learning and motivation 2", Academic Press, pages 47 to 89; Baddeley A. November 2000,"The episodic buffer: a new component of working memory?", Trends Cogn. Sci. (Regul. Ed.) 4 (11), pages 417 to 423). Working memory tasks are considered to require monitoring (i.e., manipulation of information or behaviors) as part of completing goal-directed actions in the setting of interfering processes and distractions. The cognitive processes needed to achieve this include the executive and attention control of short-term memory, which permit interim integration, processing, disposal, and retrieval of information. These processes have been found to be sensitive to age.

It is also well accepted that short-term memory is the capacity for holding a small amount of information in mind in an active, readily available state for a short period of time. The duration of short-term memory is believed to be in the order of seconds. A commonly cited capacity is 7 ± 2 elements which can be simultaneously kept in the short-term memory (Miller G., March 1956, "The magical number seven plus or minus two: some limits on our capacity for processing information", Psychological Review 63 (2): 1-979). In contrast, long-term memory can hold an indefinite amount of information.

Working memory is mandatory for everyday life and is reduced in aging (Gazzaley A. et al. Nature Neuroscience 8, 1298- 1300 (2000)). Apart from aging working memory deficits can be caused by underlying dementias which are commonly ascribed to central executive impairment and assumed to relate to frontal lobe dysfunction. Working memory is reduced in Parkinson's disease.

Patients suffering from dementia of the Alzheimer type (AD) are particularly impaired in the functioning of the central executive component of working memory. Performance failures on standard tests of attention and executive function reinforce this interpretation. Alzheimer's disease (AD) is the leading cause of dementia, there are 35 million people suffering from this progressive neurodegenerative disorder today. The number of patients with dementias will double by 2030 and it is estimated that more than 115 million people with brain disorder by 2050 will be affected. According to the Alzheimer's Association, AD is the third most expensive disease ranking after cardiovascular diseases and cancer. There is no cure and early preclinical diagnostic assay available for AD. Currently there is only symptomatic therapy available, which is not able to stop progression of the disease. Global costs of dementia (social care and medical costs) are currently more than 1 % GDP and are likely to increase 85% by 2030. Donepezil, rivastigmine and galantamine - are known as cholinesterase inhibitors. Cholinesterase inhibitors, particularly rivastigmine and donepezil, may reduce the severity of some behavioural and psychological symptoms in dementia and help delay their onset of dementia. Cholinesterase inhibitors are especially effective in Lewy body dementia and dementia related to Parkinson's disease in treating agitation, apathy and psychotic symptoms. Memantine is a medium-affinity NMDA (N-methyl-D-aspartate) receptor antagonist. Memantine forms an independent class amongst modem anti-dementives due to its unique glutamatergic active principle, as a substance acting specifically on the glutamatergic neurotransmitter system, benzodiazepines, antidepressant, anticonvulsants and mood stabilizers. All aforementioned drugs can be used in combination.

It was also found that schizophrenics exhibit impaired spatial delayed-response tasks, in analogy to those that have been used to assess working memory function of the dorsolateral prefrontal cortex in rhesus monkeys. In this study schizophrenic patients and two control groups, normal subjects and bipolar psychiatric patients were tested on the oculomotor version of the memory task, a haptic version of the same task and two control tasks: i) a sensory task that did not require working memory and ii) a digit span test. Patients with schizophrenia showed marked deficits as compared to the two control groups (Sohee Park et al., Arch Gen Psychiatry, 1992, 49(12), 975 to 982). It was shown that spatial working memory in participants with childhood-onset schizophrenia and attention-deficit/hyperactivity disorder (ADHD) showed deficits in verbal and spatial working memory (Canan K. et al., Psychiatry Research, Volume 80, Issue 2, 17 August 1998, pages 165 to 176).

Although numerous attempts have been made an efficient and effective way of how to cope with impairment of working memory or short term memory deficits has not been found yet.

In WO 03/059873 A1 pharmaceutical compounds are disclosed comprising both a thiazole group and a quaternary carbon comprising three phenyl groups next to a sulfoxide moiety.

These compounds shall furnish a medicament which regulates calcium-activated potassium channels and which acts as modulator of SKca and/or IKca channels. According to this document the proposed Pharmaceutical compounds shall be suited for the treatment of diseases or conditions like respiratory diseases such as asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary heart disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type 11, hyperinsulinemia, premature labour, baldness, cancer, and immune suppression in particular in for reducing or inhibiting undesired immune-regulatory actions, Addison's disease, alopecia areata, Ankylosing spondylitis, haemolytic anemia (anemia haemolytica), pernicious anemia (anemia perniciosa), aphthae, aphthous stomatitis, arthritis, arteriosclerotic disorders, osteoarthritis, rheumatoid arthritis, aspermiogenese, asthma bronchiale, auto-immune asthma, auto-immune hemolysis, Bechet's disease, Boeck's disease, inflammatory bowel disease, Burkitt's lymphom, Chron's disease, chorioiditis, colitis ulcerosa, Coeliac disease, cryoglobulinemia, dermatitis herpetiformis, dermatomyositis, irrsulin-dependent type I diabetes, juvenile diabetes, idiopathic diabetes insipidus, insulin-dependent diabetes mellisis, auto- immune demyelinating diseases, Dupuytren's contracture, encephalomyelitis, encephalomyelitis allergic, endophthalmia phacoanaphylactica, enteritis allergic, auto-immune enteropathy syndrome, erythema nodosum leprosum, idiopathic facial paralysis, chronic fatigue syndrome, febris rheumatica, glomerulo nephritis, Goodpasture's syndrome, Graves'disease, Hamman-Rich's disease, Hashimoto's disease, Hashimoto's thyroiditis, sudden hearing loss, sensoneural hearing loss, hepatitis chronica, Hodgkin's disease, haemoglobinuria paroxysmatica, hypogonadism, ileitis regionalis, iritis,. leucopenia, leucemia, lupus erythematosus disseminatus, systemic lupus erythematosus, cutaneous lupus erythematosus, lymphogranuloma malignum, mononucleosis infectiosa, myasthenia gravis, traverse myelitis, primary idiopathic myxedema, nephrosis, ophthalmia symphatica, orchitis granulomatosa, pancreatitis, pemphigus, pemphigus vulgaris, polyarteritis nodosa, polyarthritis chronica primaria, polymyositis, polyradiculitis acuta, psoreasis, purpura, pyoderma gangrenosum, Quervain's thyreoiditis, Reiter's syndrome, sarcoidosis, ataxic sclerosis, progressive systemic sclerosis, scleritis, sclerodermia, multiple sclerosis, sclerosis disseminata, acquired spenic atrophy, infertility due to antispermatozoan antobodies, thrombocytopenia, idiopathic thrombocytopenia purpura, thymoma, acute anterior uveitis, vitiligo, AIDS, HIV, SCID and Epstein Barr virus associated diseases such as Sjorgren's syndrome, virus (AIDS or EBV) associated B cell lymphom, parasitic diseases such as Lesihtnania, and immunosuppressed disease states such as viral infections following allograft transplantations, graft vs. Host syndrome, transplant rejection, or AIDS, cancers, chronic active hepatitis diabetes, toxic chock syndrome, food poisoning, and transplant rejection.

US 2002/0183334 is about substituted thioacetamides. The rather broad set of compounds disclosed in US 2002/0183334 shall be suited for the treatment of sleepiness, tiredness, Parkinson's disease, cerebral ischemia, stroke, sleep apneas, eating disorders, attention deficit hyperactivity disorder, cognitive dysfunction, disorders associated with hypofunctionality of the cerebral cortex, depression, schizophrenia, and chronic fatigue syndrome, and also for the promotion of wakefulness, stimulation of appetite, or stimulation of weight gain.

In WO 03/066035 a broad range of thioether sulfonamides is disclosed which shall be suited for the treatment of diabetes mellitus.

US 2005/0234040 refers to tricyclic aromatic and bis-phenyl sulfinyl derivatives. The compounds specified in this document shall furnish medicaments suited for the treatment of excessive sleepiness, promotion and/or improvement of wakefulness, preferably improvement of wakefulness in patients with excessive sleepiness associated with narcolepsy, sleep apnea, preferably obstructive sleep apnea/hypopnea, and shift work disorder), Parkinson's disease, Alzheimer's disease, cerebral ischemia, stroke, eating disorders, attention deficit disorder ("ADD"), attention deficit hyperactivity disorder ("ADHD"), depression, schizophrenia, fatigue, preferably fatigue associated with cancer or neurological diseases, such as multiple sclerosis and chronic fatigue syndrome, stimulation of appetite and weight gain and improvement of cognitive dysfunction.

Compounds and methods which are specifically designed for the improvement of short term and/or working memory are still missing. It is therefore an object of the present invention to provide agents that increase short term memory and/or working memory performance, in particular in both impaired as well as in healthy subjects. It is another object of the present invention to treat age-dependent decline of working memory and/or short term memory and to provide an effective agent which can be used for the treatment of disorders that in turn require improvement of working memory and/or short term memory.

Therefore, the present invention provides a chemical compound having the general formula (I): 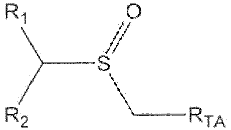 wherein R₁ and R₂ are, equal or independently, aryl, heteroaryl, fused aryl, fused heteroaryl, mono or multi-substituted aryl, mono or multi-substituted heteroaryl, mono or multi-substituted fused aryl, mono or multi-substituted fused heteroaryl;
R_{TA} is a 2-1,3-, or 4-1,3- or 5-1,3-thiazole ring with the general Formula (IIa) or a 1,3,4-thiadiazole ring with the general Formula (IIb) or a 1,3,4-oxadiazole ring with the general Formula (IIc), 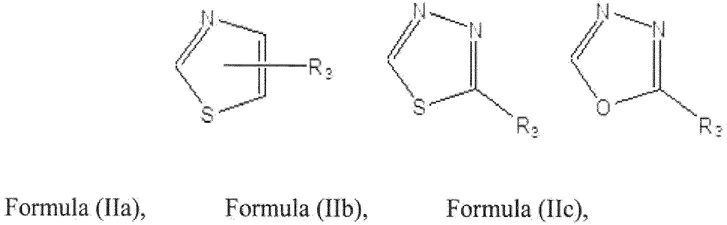 R₃ is present 1 or 2 times, equal or independently,
wherein R₃ is selected from the group consisting of hydrogen, alkyl, cycloalkyl, heterocycloalkyl, alkylamino, in particular dialkylamino, arylamino, in particular diarylamino, hydroxyalkylamino, alkoxy, arylalkyl, hydroxyalkyl, thioalkyl, haloalkyl, haloaryl, haloarylalkyl, haloalkoxy, mono or multi-substituted alkyl, mono or multi-substituted cycloalkyl, mono or multi-substituted heterocycloalkyl, mono or multi-substituted alkylamino, in particular dialkylamino, mono or multi-substituted arylamino, in particular diarylamino, mono or multi-substituted hydroxyalkylamino, mono or multi-substituted alkoxy, mono or multi-substituted arylalkyl, mono or multi-substituted hydroxyalkyl, mono or multi-substituted thioalkyl, mono or multi-substituted haloalkyl, mono or multi-substituted haloaryl, mono or multi-substituted haloarylalkyl, mono or multi-substituted haloalkoxy and carboxylate ester, wherein substituted means substituted with a residue selected from the group consisting of alkyl, cykloalkyl, heterocycloalkyl, hydroxyalkyl, alkylthio, ether, hydroxyl, fluoride, chloride, bromide and iodide.

It has been found that those compounds according to formula (I) are preferred in which R3 is hydrogen or alkyl, in particular methyl, or hydroxyalkyl, in particular hydroxymethyl. Those compounds according to formula (I) are also preferred in which R3 is alkyl substituted with at least one residue selected from the group consisting of heterocycloalkyl, carboxylic acid, amide and ester. Even more preferred in this regard are R3 residues in which alkyl is methyl substituted with heterocycloalkyl, preferably heterocycloalkyl containing at least two heteroatoms. That is, in this embodiment alkyl respresents an alkylene bridging group, in particular a methylene bridging group, between the heterocycle according to formula (IIa), (IIb) or (IIc) and the heterocycloalkyl group. Heterocycloalkyl is preferably selected from the group consisting of morpholinn, piperazino and methylpiperazino.

Aryl, i.e.an aryl group in the meaning of the present invention preferably denotes phenyl, and substituted aryl preferably denotes mono or multi-substituted phenyl. Exemplary substituted phenyl include for example -o-C₆H₄-R', -m-C₆H₄-R' and -p-C₆H₄-R', wherein R' is defined as the residue R3 above for formula (I). Fused aryl in the meaning of the present invention preferably denotes an aromatic ring being fused with at least one aromatic ring system, the fused aryl group in particular being made of five to 15 carbon atoms, e.g. naphthyl and anthracenyl. These fused aryl may also be mono or multi-substituted, e.g. 1-naphthyl, 2-naphthyl, 1-anthracenyl or 2-anthracenyl, as defined herein.

Heteroaryl, i.e. a heteroaryl group in the meaning of the present invention preferably denotes a 3 to 8-membered heterocyclic aromatic group which contains at least one heteroatom selected from the group consisting of O, N, and S. Heteroaryl in the meaning of the present invention also includes a heteroaryl ring being fused to another aromatic ring. Both heteroaryl and fused heteroaryl group can also be mono or multi-substituted as defined herein.

Alkyl, i.e. an alkyl group in the meaning of the present invention preferably comprises an alkyl, alkenyl and alkynyl residue, in particular a C₁-C₆-alkyl, C₂-C₆-alkenyl and C₂-C₆-alkynyl residue. Suitable residues are selected from the group consisting of -CH₃, -C₂H₅,-CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃,-C(R')₃, -C₂(R')₅, -CH₂-C(R')₃,-C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-CH₃H₇, -C₂H₄-CH=CH-CH₃, -CH₂-CH=CH-C₂H₅, -CH₂-CH=CH-CH=CH₂, -CH=CH-CH-CH-CH₃, -CH=CH-CH₂-CH=CH₂, -C(CH₃)=CH-CH=CH₂, -CH-C(CH₃)-CH=CH₂,-CH=CH-C(CH₃)=CH₂,-CH₂-CH-C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH-CH₂, -CH=C(CH₃)-CH=CH₂, -CH=CH-C(CH₃)-CH₂, -G(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, and -C₂H₄-C≡C-C₂H₅;
wherein R' can be defined as residue R3 as outlined above for formula (I). R' as referred to above preferably is hydrogen, methyl, ethyl, n-propyl, i-propyl, n-butyl, tert-butyl, sec-butyl or isobutyl, in particular hydrogen, methyl or i-propyl.

The alkyl group according to one preferred embodiment of formula (I), in particular for residue R3, is methyl, ethyl, propyl, isopropyl, n-butyl, tert-butyl, sec-butyl, and isobutyl, preferably methyl or isopropyl, and more preferably alkyl is selected from the group consisting of methyl, ethyl, iso-propyl, and tert-butyl.

Arylalkyl, i.e. a arylalkyl group in the meaning of the present invention preferably denotes a linear or branched C₁-C₆-alkyl substituted with at least one aryl group as defined above, preferably benzyl or phenylethyl. Suitable mono or multi-substituted arylalkyl comprise for example 4-hydroxybenzyl, 3-fluorobenzyl or 2-fluorophenylethyl.

Cycloalkyl, i.e. a cycloalkyl group in the meaning of the present invention preferably denotes a non-aromatic ring system containing three to eight carbon atoms, preferably four to eight carbon atoms. With mono or multi-substituted cycloalkyl one or more of the carbon atoms in the ring may be substituted by a group R' as defined above, preferably methyl, ethyl, n-propyl, i-propyl, n-, i- or t-butyl. Suitable C₃-C₈-cycloalkyl groups can be selected from the group consisting of -cyclo-C₃H₅, -cyclo-C₄H₇, -cyclo-C₅H₉, -cyclo-C₆H₁₁, -cyclo-C₇H₁₃, and -cyclo-C₈H₁₅.

Heterocycloalkyl, i.e. heterocycloalkyl group in the meaning of the present invention preferably denotes a non-aromatic carbon ring system in which one or more ring carbon atoms have been replaced by a heteroatom functional group such as O, S, SO, SO₂, N, or NR'2, wherein R' is defined as outlined above. Preferred heterocycloalkyl groups can be selected from the group consisting of morpholine-4-yl, piperazinyl, and I-alkylpiperazine-4-yl.

Hydroxyalkyl, i.e. a hydroxyalkyl, group in the meaning of the present invention preferably denotes a hydroxyalkyl group in which the alkyl group is defined as outlined above, preferable methyl, ethyl, n-propyl, i-propyl, n-, i- or t-butyl. Suitable hydroxyalkyl groups are selected from the groups consisting of a hydroxymethyl, hydroxyethyl, hydroxypropyl, and hydroxy-iso-propyl group. The hydroxyalkyl group according to a preferred embodiment of formula (I) is hydroxymethyl. Once bonded the alkyl or methyl group may also be denoted as alkylene or methylene group, respectively.

Alkylthio, i.e. an alkylthio group in the meaning of the present invention preferably denotes an alkylthio group in which the alkyl group is defined as outlined above, preferably for R3, in particular thiomethyl.

Haloalkyl, i.e. a haloalkyl group in the meaning of the present invention preferably denotes a haloalkyl group in which the alkyl group is defined as outlined above, in particular for R3, and wherein said alkyl is substituted by one or more halogen atoms, preferably substituted by one to five halogen atoms, in particular by floor or chlorine atoms. In a preferred embodiment the haloalkyl, group is selected from the group consisting of -C(R¹⁰)₃, - CR¹⁰(R^{10'})₂, -CR¹⁰(R^{10'})R^{10"}, -C₂(R¹⁰)₅, -CH₂-C(R¹⁰)₃, -C(R^{10'})₂-CH(R^{10'})₂, -CH₂-CR¹⁰(R^{10'})₂, -CH₂-CR¹⁰(R^{10'})R^{10"}, -C₃(R¹⁰)₇, or -C₂H₄-C(R¹⁰)₃, wherein R¹⁰, R^{10'}, R^{10"} represent F, Cl, Br or I, preferably F.

Haloaryl, i.e. a haloaryl group in the meaning of the present invention preferably denotes a haloaryl group in which at least one aromatic carbon atom is substituted by one or more halogen atoms, preferably substituted by one to five halogen atoms. Preferably, halogen atoms are selected from the group consisting of fluorine, chlorine and bromine atoms, and preferably fluorine.

Haloarylalkyl, i.e. a haloarylalkyl group in the meaning of the present invention preferably denotes a haloarylalkyl group in which the arylalkyl group is defined as outlined above and wherein at least one aromatic carbon atom of said aryl group is substituted by one or more halogen atoms, preferably substituted by one to five halogen atoms. Preferably, halogen atoms are selected from the group consisting of fluorine, chlorine, bromine and iodine atoms, more preferably from fluorine and chlorine atoms.

Haloalkoxy, i.e. a haloalkoxy group in the meaning of the present invention preferably denotes a haloalkoxy group in which the alkoxy group is defined as outlined above and wherein said alkoxy is substituted by one or more halogen atoms, preferably substituted by one to five halogen atoms, in particular by fluorine, chlorine, bromine and/or iodine atoms, more preferably fluorine and/or chlorine atoms. Preferably, the haloalkoxy group is selected from the group consisting of -OC(R¹⁰)₃, -OCR¹⁰(R^{10'})₂, -OCR¹⁰(R^{10'})R^{10"}, -OC₂(R¹⁰)₅,-OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F.

Alkylamino, i.e. an alkylamino group in the meaning of the present invention preferably denotes an alkylamino group in which the alkyl group is defined as outlined above, in particular for R3. The alkylamino group in the meaning of the present invention preferably denotes a (alkyl)₂-N-group, i.e. a dialkylamino group, or a alkyl-NH- group. The dialkylamino group is preferred. Alkyl is preferably defined as outlined above.

Arylamino, i.e. a diarylamino group in the meaning of the present invention preferably denotes an arylamino group in which the aryl group is defined as outlined above. The arylamino group denotes a (aryl)₂-N-group, i.e. a diarylamino, group, or a aryl-NH- group. The diarylamino group is preferred, in particular the diphenylamino group. Aryl is preferably defined as outlined above.

Hydroxyalkylamino, i.e. an hydroxyalkylamino group in the meaning of the present invention preferably denotes an hydroxyalkylamino group in which the alkyl group is defined as outlined above, in particular for R3, and wherein the hydroxyalkylamino group denotes a (HO-alkyl)₂-N-group or a HO-alkyl-NH- group, or a (HO-alkyl)-N-(alkyl)- group. Alkyl is preferably defined as outlined above.

Those compounds according to formula (I) are particularly preferred in which at least one of R₁ and R₂ is aryl or substituted aryl, preferably phenyl or substituted phenyl, e.g. tolyl.

According to another embodiment of the compound of formula (I) R₁ is aryl or substituted aryl, preferably phenyl or substituted phenyl, e.g. tolyl, and R₂ is heteroaryl or substituted heteroaryl.

It has been found that most promising results can be obtained when in formula (I) both R₁ and R₂ are phenyl or substituted phenyl, preferably phenyl.

Moreover, those compounds according to formula (I) furnish particularly improved results in terms of cognitive functions such as short term memory and/or working memory, in particular working memory, in which R_{TA} is a thiazole group, preferably a 2-thiazole, group, especially 2-thiazole, or a 4-thiazole group, in particular 2-methyl-4-thiazole. In another preferred embodiment R_{TA} is a thiadiazole group, preferably a 1,3,4-thiazole-5-oxyalkyl group, especially 1,3,4-thiadiazole-5-oxymethyl. And, in another preferred embodiment of the compound according to formula (I) R_{TA} is a 1,3,4-oxadiazole group, preferably 1,3,4-oxadiazole.

Preferred compounds according to formula (I) in terms of improving cognitive functions, in particular short term memory and/or working memory, more preferably working memory, are those selected from the group consisting of 2-(diphenylmethanesulfinylmethyl)-1,3-thiazole, 4-(diphenylmethanesulfinylmethyl)-1,3-thiazole, 2-(diphenylmethanesulfinylmethyl)-4-methyl-1,3-thiazole, 4-(diphenylmethanesulfinylmethyl)-2-methyl-1,3-thiazole, 2-(diphenylmethanesulfinylmethyl)-4-oxymethyl-1,3,4,-thiadiazole, and 2-(diphenylmethanesulfinylmethyl)-1,3,4-oxadiazole, preferably 4-(diphenylmethanesulfinylmethyl)-2-methyl-1,3-thiazole. Particularly preferred in this context, particularly with regard to working memory improvement, is 4-(diphenylmethanesulfinylmethyl)-2-methyl-1,3-thiazole.

Accordingly, with the compounds according to formula (I), preferably containing the group of the general formula (IIa) or (IIb), in particular (IIa), a novel chemotype of compounds, in particular with working memory enhancing effects, is provided with the present invention that can be used for improving cognitive functions, in particular short term memory and/or working memory, more preferably working memory, both with healthy, e.g. healthy aging, and impaired, e.g. impaired aging, individuals. Moreover, with the compounds of formula (I) also patients with dementing disorders where working memory and/or short term memory enhancing is of benefit can be effectively treated. That is, with the compounds of the present invention as specified with formula (I), preferably containing the group of the general formula (IIa) or (IIb), in particular (IIa), cognitive functions and in particular the working memory in human individuals, in particular in aging individuals can be improved. Hence, the present invention provides a compound according to formula (I) for use in improving cognitive functions and in particular the short term memory and/or working memory, in particular working memory, in human individuals, in particular in aging individuals. However, in general with the present invention compounds of formula (I) as defined herein are provided for use in therapy, i.e. for use as a medicament.

It is of particular interest that with the compounds of formula (I), preferably containing the group of the general formula (IIa) or (IIb), in particular (IIa), improvements in terms of cognitive functions, in particular short term memory and/or working memory, can be accomplished with human individuals who do not have short term memory deficits and/or working memory deficits and/or cognitive defects. Accordingly, these results can be obtained with individuals who do not have short term memory deficits and/or working memory deficits and/or cognitive defects caused by diseases of the brain. The compounds according to the present invention can thus also be administered to and are also effective with healthy individuals to improve short term memory and/or working memory, in particular working memory, and/or other cognitive abilities (or to overcome respective deficits). Accordingly, the present invention therefore also relates to the use of a compound according to the present invention for the improvement of short term memory and/or working memory, in particular working memory, and/or cognitive deficits in healthy individuals, in particular in healthy aging individuals.

In addition, with the compounds of formula (I), preferably containing the group of the general formula (IIa) or (IIb), in particular (IIa), improvements in terms of cognitive functions, in particular short term memory and/or working memory, more preferably working memory, can also be accomplished with human individuals who have short term memory deficits and/or working memory deficits and/or cognitive defects, in particular working memory deficits, caused by diseases of the brain. In this regard, a patient can be treated with the compounds according to formula (I), preferably containing the group of the general Formula (IIa) or (IIb), in particular (IIa), who has short term memory deficits and/or working memory deficits and/or cognitive defects caused by Alzheimer; Down syndrome; vascular cognitive impairment, stroke; frontotemporal dementia; behavioural, semantic or progressive aphasia type dementia; dementia with Lewy bodies; subcortical dementias; Parkinson's disease dementia; alcohol related dementia; dementia caused by traumatic brain injury; Huntington's disease related dementia; AIDS-related dementia; attention deficit disorders; working memory deficiencies related to ageing; working memory disorders related to viral infections of the brain; or schizophrenia, or who has any form of cognitive impairment, in particular working memory impairments.

The compounds according to formula (I) of the present invention can be used alone or in combination with other cognitive enhancing compounds such as rivastigmin, donepezil, galanthamin, clozapine, risperidone, memantine, olazapine, aripiprazole, quitiapine, clozapine, D1 agonists, nicotinic alfa 7-agonists, d-serine, d-cycloserine. PDE2, 4,9 inhibitors, AMPA agonists, lamotrigine, N-desmethylclozapine, mGlu receptor agonists, GABA A receptor agonists, muscarinic 1 and 4 receptor agonists, to treat cognitive impairments and improve working memory and/or short term memory, in particular working memory.

The compounds according to the present invention are particularly useful for treating patients having working memory deficits, short term memory deficits and/or cognitive defects due to particular demands in healthy and aging individuals, diseases of the brain, mental disorders or cognitive deficits, especially patients having Alzheimer, Down syndrome, stroke (vascular cognitive impairment), frontotemporal dementia, behavioural, semantic and progressive aphasia type dementia, dementia with Lewy bodies, subcortical dementias, and Parkinson's disease dementia, alcohol related dementia, dementia caused by traumatic brain injury, Huntington's disease related dementia, AIDS-related dementia, attention deficit disorders; working memory deficiencies related to ageing working memory disorders related to viral infections of the brain; or schizophrenia.

The compound according to formula (I), preferably containing the group of the general Formula (IIa) or (lib), in particular (IIa), can be administered to a patient having or being at risk to develop any of the disorders referred to above in an effective amount. It is also pos-possible to administer the compound to healthy individuals for improving working memory, short term memory and/or cognitive ability, in particular for improving working memory. According to a preferred embodiment, it is convenient to administer the compound according to the present invention by a single dosage unit form, especially as a capsule or a tablet. A preferred formulation comprises a soft gelatine capsule containing at least one compound according to formula (I) dissolved in oil within the capsule with one or more emulsifying agents. Accordingly, gelatine capsules are preferred which comprise one or more emulsifying agents and also at least one compound according to formula (I) dissolved in at least one oil.

Preferred dosages comprise administration dosages of about 1 mg/kg to about 10 mg/kg body weight. Suitable dosage forms also comprise formulations comprising at least one compound according to formula (I) in an amount of about 0.1 mg to about 10 g, preferably of about I mg to about I g, and more preferably of about 10 mg to about 200 mg. According to a preferred embodiment, the compound of formula (I) according to the present invention can be part of formulations which contain at least or can be administered with one or more compounds, also referred to as co-agents, selected from the group consisting of Rivastigmin, Donepezil, Galanthamin, clozapine, risperidone, memantine, olanzapine, aripiprazole, quitiapine, clozapine, D1 agonists, nicotinic alfa 7, agonists, d-serine, d-cycloserine, PDE2,4,9 inhibitors, AMPA agonists, Lamotrigine, N-desmethylclozapine, mGlu receptor agonists, GABA A receptor agonists, and Muscarinic 1 and 4 agonists or other potential cognitive enhancing compounds.

The object of the present invention has also been solved by a use of a compound according to formula (I) in the manufacture of a medicament for the improvement of cognitive functions in human individuals, in particular for the improvement of the short term memory and/or of the working memory, in particular working memory.

Moreover, the object of the present invention has also been solved by a pharmaceutical preparation, also referred to herein by pharmaceutical composition, comprising at least one compound according to formula (I) and at least one pharmaceutically acceptable carrier and/or diluent. Thus, according to another aspect, the present invention relates to a pharmaceutical preparation comprising a compound according to the present invention and a pharmaceutically acceptable carrier or diluent.

Pharmaceutical "carriers", "diluents" as well as "excipients" are substances to be admixed in a pharmaceutical composition as auxiliary substances to the compound according to the present invention or other compounds with pharmaceutical effect (e.g. that have also neurological or behavioural effects). These auxiliary substances do not have a pharmaceutical effect on their own (they are no "active substance" per se) but may assist to optimise the effectivity of the compound according to the present invention. The substances used as "carriers", "diluents" and excipients" can often be used interchangeably (i.e. that substances that are used as "diluents" may also serve as "excipients" and/or "carriers").

In general, "carriers" are substances that improve the delivery and the effectiveness of drugs; "diluents" are substances that dilute the active substance in a pharmaceutical preparation; "excipients" are substances that are admixed to the pharmaceutical formulation for defining its releasing properties.

All such "carriers", "diluents" and "excipients" (i.e. auxiliary substances, sometimes also the term "excipient" is used as the general term including all such substances) are, as already stated, inactive substances formulated alongside the active ingredient ("API") of a medication, for the purpose of bulking-up formulations that contain potent active ingredients (thus often also referred to as "bulking agents", "fillers", or "diluents"). Bulking up allows convenient and accurate dispensation of a drug substance when producing a dosage form. They also can serve various therapeutic-enhancing purposes, such as facilitating drug absorption or solubility, or other pharmacokinetic considerations. Such substances can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients, carriers and diluents also depends upon the route of administration and the dosage form, as well as the active ingredient and other factors. Such substances e.g. serve as antiadherents, binders, coatings, disintegrants, fillers, flavours, colours, lubricants, glidants, sorbents, preservatives, sweeteners, etc.

Drug "carriers" are substances that serve as mechanisms to improve the delivery and the effectiveness of drugs. Drug carriers are usually used in sundry drug delivery systems such as: controlled-release technology to prolong in vivo drug actions; decrease drug metabolism, and reduce drug toxicity.

Carriers are generally also used in designs to increase the effectiveness of drug delivery to the target sites of pharmacological actions.

Drug "diluents" are usually substances that simply dilute or reconstitute a pharmaceutical composition (e.g. after storage in dry form).

The pharmaceutical composition with the at least one compound according to formula (I) usually comprises said compound in an amount of about 0.01 (%w/w) to about 80 (%w/w) in a solid composition or about 0.001 (%w/v) to about 80 (%w/v) in a liquid composition. Preferably the compound according to the present invention is present in an amount of about 0.1 (%w/w) to about 50 (%w/w) in a solid composition or about 0.01 (%w/v) to about 10 (%w/v) in a liquid composition, especially of about 1 (%w/w) to about 0 (%w/w) in a solid composition or about 0.1 (%w/v) to about 5 (%w/v) in a liquid composition.

As already stated, preferably, the compounds according to formula (I) are provided as a pharmaceutical composition, especially as a pharmaceutical single unit dosage form. Pharmaceutical compositions and single unit dosage forms provided herein comprise a prophylactically or therapeutically effective amount of one or more compounds according to formula (I) and optionally also one or more additional prophylactic or therapeutic agents (e.g., in particular a compound provided herein, or other prophylactic or therapeutic agent), and one or more pharmaceutically acceptable carriers or excipients or diluents. In a specifically preferred embodiment and in this context, the term "pharmaceutically acceptable" means approved by a regulatory agency of the Federal or a state government or listed in the European or U.S. Pharmacopoeia or other generally recognized pharmacopeia for use in animals, and more particularly in humans.

The term "carrier" refers in one embodiment to a diluent, adjuvant (e.g., Freund's adjuvant (complete and incomplete)), or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a particular carrier when the pharmaceutical composition is administered intravenously. Saline solutions and aqueous dextrose and glycerol solutions can also be employed as liquid carriers, particularly for injectable solutions.

In one embodiment, Pharmaceutical compositions and dosage forms comprise one or more excipients. Suitable excipients are well-known to those skilled in the art of pharmacy, and non-limiting examples of suitable excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form usually depends on a variety of factors well known in the art including, but not limited to, the way in which the dosage form will be administered to a patient and the specific active ingredients in the dosage form. The composition or single unit dosage form, if desired, can also contain minor amounts of wetting or emulsifying agents, or pH buffering agents.

Examples of dosage forms include, but are not limited to: tablets; chaplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

Examples of suitable excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatine, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions provided herein is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVlCEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Disintegrants can be used in the compositions of the present invention to provide solid dosage forms such as tablets that disintegrate when exposed to an aqueous environment.

Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms provided herein. The amount of disintegrant used varies based upon the type of formulation, and is readily discernible to those of ordinary skill in the art. Typical pharmaceutical compositions comprise from about 0.5 to about 15 weight percent af disintegrant, specifically from about 1 to about 5 weight percent of disintegrant.

Suitalbe disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Suitable lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, methyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. If used at all, lubricants are typically used in an amount of less than about I weight percent of the pharmaceutical compositions or dosage forms into which they are incorporated.

The active ingredients described herein such as the compounds according to formula (I) and/or the co-agents provided herein can be administered by controlled release means or by delivery devices that are well known to those of ordinary skill in the art. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients. Thus provided are single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood revels of the drug, and can thus affect the occurrence of side (e.g., adverse) effects.

Most controlled-release formulations are designed to initially release an amount of drug (active ingredient) that promptly producers the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

Parenteral dosage forms can be administered to patients by various routes including, but not limited to, subcutaneous, intravenous (including bolus injection), intramuscular, and intraarterial. Because their administration typically bypasses patients' natural defences against contaminants, parenteral dosage forms are in certain embodiments sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, com oil, cottonseed oil, peanut oil, sesame oil, ethyl oleate, isopropyl myristate, and benzyl benzoate.

According to a preferred embodiment, the pharmaceutical preparation of the present invention further comprises one or more compounds selected from the group consisting of Rivastigmin, Donepezil, Galanthamin, clozapine, risperidone, memantine, olanzapine, aripiprazole, quitiapine, clozapine, D1 agonists, nicotinic alfa 7, agonists, d-serine, d-cycloserine, PDE2,4,9 inhibitors, AMPA agonists, Lamotrigine, N-desmethylclozapine, mGlu receptor agonists, GABA A receptor agonists, and Muscarinic 1 and 4 agonists or other potential cognitive enhancing compounds, agents.

Preferred dosages of the compound according to formula (I) are 0.1 mg to 10 g, preferably of 1 mg to 1 g, especially 10 mg to 200 mg, of the compound according to the present invention. As already stated, the invention relates to the use of a composition according to formula (I) for the manufacture of a medicament. And, the invention also relates to the use of a composition comprising at least one compound according to formula (I) for the manufacture of a remedy i.e. pharmacological treatment or prevention of cognitive impairment, dementia and deficits of short term and/or working memory.

The compound of the formula (I) can be administered to humans, rodents other mammals, as therapeutics per se, and as mixtures with one another or in the form of pharmaceutical preparations which allow enteral or parenteral use and which as active constituent contain an effective dose of the compound of formula (I). The effective dose range can easily be adapted to the severity and the need of the individual patient. Preferred doses are from 1 mg to 10 mg/kg body weight, in addition to customary pharmaceutically innocuous excipients and additives.

The proposed therapeutics can be administered orally, e.g. in the form of pills, tablets, coated tablets, sugar coated tablets, hard and soft gelatin capsules, solutions, syrups, emulsions or suspensions. Administration, however, can also be carried out rectally, e.g. in the form of suppositories, or parenterally, e.g. in the form of injections or infusions or percutaneously, e.g. in the form of ointments, creams or tinctures.

In addition to the active compound of formula (I), the pharmaceutical composition can contain further customary, usually inert carrier materials or excipients, in particular as outlined above. Thus, the pharmaceutical preparations can also contain additives, such as, for fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants.

The compounds of the present invention can be used in the form of one substance alone or in combination with other active compounds - for example with remedies already known to improve short term memory and/or working memory of healthy individuals, in particular of healthy aging individuals, and in particular the working memory.

The invention also provides a pharmaceutical composition comprising compounds of formula (I), in free form or in the form of pharmaceutically acceptable formulations and together with pharmaceutically acceptable diluents or carriers.

The compounds of formula (I) and also the pharmaceutically acceptable salt or solvate thereof can be used as the active principle for improvement of cognitive functions, in particular for the enhancement of short term memory and/or working memory, also in healthy or unimpaired individuals.

The compound according to the present invention is defined by formula (I), wherein the connection between the methylene group and the heterocyclic compound R_{TA} can be with any position where there is no R₃ and where there is a free valence available for the formation of a covalent bond. As well, the connection between R₃ with the heterocycle can be with any position where the connection of the methylene group is not present and where there is a free valence available for the formation of a covalent bond. R₃ is present in formulae (IIa), (IIb) and (IIc) independently 1, or 2 times, equal or independently. R₃ preferably is hydrogen or an alkyl group, in particular methyl, ethyl, iso-propyl, tert-butyl, or a hydroxyalkyl group, in particular hydroxyethyl or a cycloalkyl group, or a heterocycloalkyl group, or a dialkylamino, group, in particular dimethylamino, or a heterocycloalkyl group, in particular morpholino, piperazino or methylpiperazino.

Compounds having infinite chains consisting for instance of repeating R₁, R₂ R₃ units and the like are not encompassed by this invention.

Constituents which are optionally substituted as stated herein, may be substituted, unless otherwise noted, at any chemically possible position.

R₃ hereby may preferably not be substituted by a second or otherwise further residue selected from R₃.

The invention also provides a pharmaceutical composition comprising a compound of Formula (I), in free form or in the form of pharmaceutically acceptable salts and/or physiologically functional derivatives, together with a pharmaceutically acceptable diluent or carrier therefore. The present invention also relates to a compound of Formula (I) and physiologically acceptable salts or physiologically functional derivatives thereof. The present invention also relates to a compound of Formula (I).

In addition, the present invention provides methods for preparing the desired compounds: Such a method uses procedures from different publications (Jae-Chul Jung et al. Molecules 2012, 17, 10446-10458; WO 2005028428 A1, Jianjing Cao, ACS Med Chem Lett. 2010 October 10; 2(1): 48-52; US 7,297,817 B2, US 7,449,481 B2, US 7,314,875 B2; Faming Zhuanli Shenqing Gongkai Shuomingshu, CN 2005-10049330 20050310, 2006; Prisinzano, T. et al., Tetrahedron Asymm. 2004, 15, 1053-1058; EP 1 260 501 A1, CN 2006/10155494 20061227, 2007; WO 2005/042479 A1. The compound according to formula (I) can be obtained by the methods described herein or variations thereof in the racemic form.

Several processes are available for the preparation of compounds of Formula (I). These methods comprise the step of reacting benzhydrole with thioacetic acid or in particular of thioglycolic acid, converting the carboxylic acid into an amide then into a thionamide, then into a thiazole. The thiazole can be converted by oxidation into the racemic compound of the Formula (I).

Alternatively, it is also possible to start from benzhydrol, converting it to the thiol, reacting it with a chloromethyl-heterocycle and oxidizing it with hydrogenperoxyde.

For example 2-(diphenylmethanesulfinylmethyl)-1,3-thiazole was synthesized by condensing benzhydrole and mercaptoacetic acid with trifluoroacetic acid (TFA). Converting the acid to the acid chloride with thionylchloride and to the amide with ammonia. The thiazole was formed with 1,2-dichlaro-1-ethoxyethane and the resulting sulfide oxidize to the sulfone with hydrogenperoxyde.

In another example, benzhydrol was converted to the thiol with Lawssons reagent and then alkylated with a chloromethyl-heterocycle. The resulting sulfide was converted to the sulfoxyde with hydrogen peroxyde.

Accordingly, the compounds of the present invention can be prepared by use of a process comprising reacting diarylmethanol, in particular benzhydrol, with Lawesson's reagent, treating the reaction product diarylmethanethiol with a cloromethyl-thiazole, -thiadiazole or -oxadiazole derivative, in particular with 2-(chloromethyl)-1,3-thiazole, 4-(chloromethyl)-1,3-thiazole, 2-(chloromethyl)-4-methyl-1,3-thiazole, 4-(chloromethyl)-2-methyl-1,3-thiazole, 2-(chloromethyl)-4-oxymethyl-1,3,4-thiadiazole, or 2-(chloromethyl)-1,3,4-oxadiazole, and oxidizing the ((diarylmethyl)sulfanyl)methyl-thiazole, -thiadiazole or -oxadiazole derivative to the corresponding diarylmethanesulfinylmethyl compound.

Alternatively, the compounds of the present invention can be prepared by a process comprising reacting diarylmethanol, in particular benzhydrol, with thioglycolic acid to form 2-(diarylmehtylthio) acetic acid, converting said acetic acid derivative to 2-(diarylmethylthio) acetamide, converting said acetamide derivative, in particular by treatment with Lawesson's reagent, to the corresponding ethanethioamide derivative, converting said ethanethioamide derivative to a (((diarylmethyl)sulfanyl)methyl) thiazole, derivative, in particular to 2-(diphenylmethanesulfanylmethyl)-1,3-thiazole, 4-(diphenylmethanesulfanylmethyl)-1,3-thiazole, 2-(diphenylmethanesulfanylmethyl)-4-methyl-1,3-thiazole or 4-(diphenylmethanesulfanylmethyl)-2-methyl-1,3-thiazole, and oxidizing said thiazole, compound to the corresponding (diarylmethanesulfinylmethyl) thiazole derivative.

The present invention also contemplates a pharmaceutical composition comprising a compound of the general Formula (I) in free form and physiologically functional derivatives, together with pharmaceutically acceptable diluents or carriers.

The present invention also relates to the use of the compound of the Formula (I) and physiologically functional derivatives in the manufacture of a medicament for the cognitive enhancement of healthy individuals or aging individuals or of individuals with cognitive impairments, or for the treatment of a disease or a therapeutic indication in which improvement of cognitive functions, in particular of the working memory is beneficial.

Preferably, the pharmaceutical composition according to the present invention comprises a compound as defined by Formula (I) in free form and a pharmaceutically acceptable diluent or carrier for use in the treatment of healthy individual or aging individuals or treatment of a disease or medical condition accompanied by working memory deficits.

However, in general with the present invention also a pharmaceutical composition has been provided comprising a compound according to formula (1) and at least one pharmaceutically acceptable carrier and/or diluent, as defined herein, for use in therapy, i.e. for use as a medicament.

The present invention also relates to a pharmaceutical composition comprising a compound as defined herein in free form or in the form of a pharmaceutically acceptable diluents or carrier for use in the treatment of healthy individuals or aging individuals or for the treatment of a disease or medical condition accompanied by working memory deficits.

Accordingly, the problem of the present invention is also solved by a compound according to Formula (I) for use in improving cognitive functions in human individuals, in particular in aging individuals. Preferably, said compound for use is used in improving the short term memory and/or the working memory in human individuals, in particular for use in improving the working memory. The compound can in particular be used for improving cognitive functions, in particular short term and/or working memory, with human individual who do not have defects of cognitive functions and/or short term memory deficits and/or working memory deficits, or any such defects or deficits caused by diseases of the brain. Alternatively, the compound according to Formula (I) can be used for human individuals who have defects of cognitive functions and/or short term memory deficits and/or working memory deficits caused by diseases of the brain. The causes for such defects of cognitive functions and/or short term memory deficits and/or working memory deficits are those as mentioned above.

Accordingly, the problem of the present invention is also solved by the use of a compound according to Formula (I) for the preparation of a medicament for improving cognitive functions in human individuals, in particular in aging individuals. Preferably, said prepared medicament is used in improving the short term memory and/or the working memory in human individuals, in particular for use in improving the working memory. Said prepared medicament can in particular be used for improving cognitive functions, in particular short term and/or working memory, with human individual who do not have defects of cognitive functions and/or short term memory deficits and/or working memory deficits, or any such defects or deficits caused by diseases of the brain. Alternatively, said medicament prepared by use of the compound according to Formula (I) can be used for treating human individuals who have defects of cognitive functions and/or short term memory deficits and/or working memory deficits caused by diseases of the brain. The causes for such defects of cognitive functions and/or short term memory deficits and/or working memory deficits are those as mentioned above.

It has also surprisingly been found that the compound according to Formula (I) can be used in specifically inhibiting the dopamine transporter (DAT) in mammals, in particular human individuals, while the norepinephrine transporter (NET) and the serotonin transporter (SERT) are not or are not significantly inhibited. That is the compound according to Formula (I) has reduced or preferably essentially no activity on norepinephrine transporter (NET) and on serotonin transporter (SERT). This is preferred as activity on NET and SERT can lead to unwanted side effects. Accordingly, the present invention also is about use of the compound according to Formula (I) for improving or treating a condition in human individuals susceptible to substantially selectively inhibiting the dopamine transporter (DAT), preferably without having substantially any, or only having reduced, activity on norepinephrine transporter (NET) and/or on serotonin transporter (SERT). And, the present invention also is about use of the compound according to Formula (I) for the preparation of a medicament for improving or treating a condition in human individuals susceptible to substantially selectively inhibiting the dopamine transporter (DAT), preferably without having substantially any, or only having reduced, activity on norepinephrine transporter (NET) and/or on serotonin transporter (SERT).

"Pharmaceutically acceptable salt" refers to any salt of a compound provided herein which retains its biological properties and which is not toxic or otherwise undesirable for pharmaceutical use. Such salts may be derived from a variety of organic and inorganic counter-ions well known in the art and include: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, /ert-butylacetic, lauryl sulfuric, gluconic, benzoic, glutamic, hydroxynaphthoic, salicylic, stearic, cyclohexylsulfamic, quinic, muconic acid and the like acids; or (2) salts formed when an acidic proton present in the parent compound either (a) is replaced by a metal ion, e.g., an alkali metal ion, an alkaline earth ion or an aluminum ion, or alkali metal or alkaline earth metal hydroxides, such as sodium, potassi-potassium, calcium, magnesium, aluminum, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like. Specifically preferred salt forms are hexaflorophosphate and chloride salts.

Such salts further include, by way of example only, sodium, potassium, calcium, magnesium, ammonium, tetraalkylammonium and the like, and when the compound contains a basic functionality, salts of non-toxic organic or inorganic acids, such as hydrochloride, hydrobromide, tartrate, mesylate, besylate, acetate, maleate, oxalate and the like. The term "physiologically acceptable cation" refers to a non-toxic, physiologically acceptable cationic counterion of an acidic functional group. Such cations are exemplified by sodium, potassium, calcium, magnesium, ammonium and tetraalkylammonium cations and the like.

The compounds according to the present invention may also be provided as solvates. "Solvate" refers to a compound provided herein or a salt thereof that further includes a stoichiometric or non-stoichiometric amount of solvent bound by non-covalent intermolecular forces. Where the solvent is water, the solvate is a hydrate.

The invention is further described by the following examples given in figures, yet without being restricted thereto.
- Fig. I: shows scheme (1) for the synthesis of compounds as exemplified by 2-(diphenylmethanesulfinylmethyl}-1,3-thiazole (C1);
- Fig. 2: shows scheme (2) for the synthesis of compounds as exemplified by 4-(diphenylmethanesulfinylmethyl)-1,3-thiazole (C2);
- Fig. 3: shows H-NMR of 4-(diphenylmethanesulfinylmethyl)-1,3-thiazole (C2);
- Fig. 4: shows H-NMR of 2-(diphenylmethanesulfinylmethyl}-1,3-thiazole (C1);
- Fig. 5: shows H-NMR of 2-(diphenylmethanesulfinylmethyl)-4-methyl-1,3-thiazole (C3);
- Fig. 6: shows results of the administration of the compound C1 According to the present invention in an accepted animal model and
- Fig 7: shows the assessment of Fig. 6 including standard deviation.

### EXAMPLES

### Materials:

The following compounds were purchased:
1. Diphenylmethane (Sigma Aldrich Chemie GmbH, Germany)
2. Thioglycolic acid (Sigma Aldrich Chemie GmbH, Germany)
3. Thionylchloride (Sigma Aldrich Chemie GmbH, Germany)
4. LR, Lawesson Reagent (Sigma Aldrich Chemie GmbH, Germany)
5. Dioxane (Sigma Aldrich Chemie GmbH, Germany)
6. Trifluoroacetic acid (Sigma Aldrich Chemie GmbH, Germany)
7. Hydrogen peroxyde (Sigma Aldrich Chemie GmbH, Germany)
8. 1,2 dichloroethyl ether (ABCR GmbH & Co. KG, Germany)
9. 4-(chloromethyl)-1,3-thiazole (Sigma Aldrich Chemie GmbH, Germany)
10. Chloroacetaldehyde dimethyl acetal (MerckSchuchardt OHG, Germany)
11. Potassium tert-butoxide (Sigma Aldrich Chemise GmbH, Germany)
12. Solvents generally (Sigma Aldrich Chemie GmbH, Germany)

### A. Syntheses

### Analytics:

Abbreviations: min, minute(s); h, hour(s); r.t., room temperature; Rt, retention time; ψ, pseudo; s, singlet; t, triplet, quint, quintet; br., broad; J, coupling constant; pTLC, preparative thin layer chromatography; DMAP, 4-dimethylaminopyridine.

Analytical TLC: Merck aluminium sheets, silica gel 60 F254.

Preparative TLC: Merck PLC plates, silica gel 60 F254, 0.5 mm, 1.0 mm or 2.0 mm.

Flash chromatography: Acros silica gel 60A, 0.035 - 0.070 mm. Flash Master Personal or Flash Master II, Jones Chromatography, UK.

NMR spectra: Bruker Avance 400 MHz. The ¹H NMR spectra were recorded at 400 MHz; concentration, 1 to 5 mg/mL; temperature, 305 K. The residual solvent peaks were used as the internal standards (DMSO-d₆: δ H 2.49, δ_{C} 39.5; CDCl₃: δ_{H} 7.24, δ_{C} 77.0; CD₃OD: δ_{H} 3.30, δ_{C} 49.0). Alternatively, TMS was used as a standard (indicated with TMS).

Analytical LC/ESI-MS: Waters 2700 Autosampler. 2 x Waters 600 Multisalvent Delivery System, Waters 600 Controller. 50 µL sample loop. Column, Chromolith Speed ROD RP18e (Merck, Darmstadt), 50 x 4.6 mm, with 2 µm prefilter (Merck). Eluent A, H₂O + 0.1% HCO₂H; eluent B, MeCN. Gradient, 2 % B to 100 % B within 4 min, then isocratic for 0.90 min, then back to 2 % B within 0.15 min, then isocratic for 0.50 min; flow, 3 mL/min. Waters LCZ single quadrupol mass spectrometer with electrospray source. MS method, MS8minPM-80-800-20V; positive/negative ion mode scanning, m/z 80 - 800 or 80 - 900 in 1 s; capillary, 3.5 kV; cone voltage, 20 V; multiplier voltage, 400 V; probe and desolvation gas temperature, 120° C and 350° C, respectively. Waters 2487 Dual 280/254 nm Absorbance Detector, set to 254 nm. Software, Waters Masslynx V 4.0.

### 1. Synthesis of 4-(diphenylmethanesulfinylmethyl)-1,3-thiazole (C2)

### Mercaptobenzhydryl

To a mixture of benzhydrol (2.0g, 10.8mmol) and Lawesson's reagent (3.7g, 9.1mmo) in anhydrous dioxane (50mL) was refluxed for 3h under argon. The solvent was evaporated till dryness. The resulting oil was dissolved in DCM and washed with water. The residue was purified by flash column chromatography (hexane) to give mercaptobenzhydryl 1.36g, yield 62%.

### 4-{[(diphenylmethyl)sulfanyl]methyl}-1,3-thiazole

To a solution of thiol (2.3g, 11.5mmol) in DMF (10mL) was added potassium t-BuOK (1.68g) and 4-(chloromethyl)-1,3-thiazole (1.25g, 9.3mmol), and the reaction mixture was stirred at room temperature overnight. The next day saturated aqueous NH₄Cl was added and the reaction mixture was extracted with Et₂O. After removal of the solvent, the residue was purified by silica gel chromatography (hexane, hexane:CH₂Cl₂ 1:1) to afford 4-[(benzhydrylthio)methyl]-1,3-thiazole 5 (2g, 59%).

### 4-(diphenylmethanesulfinylmethyl)-1,3-thiazole

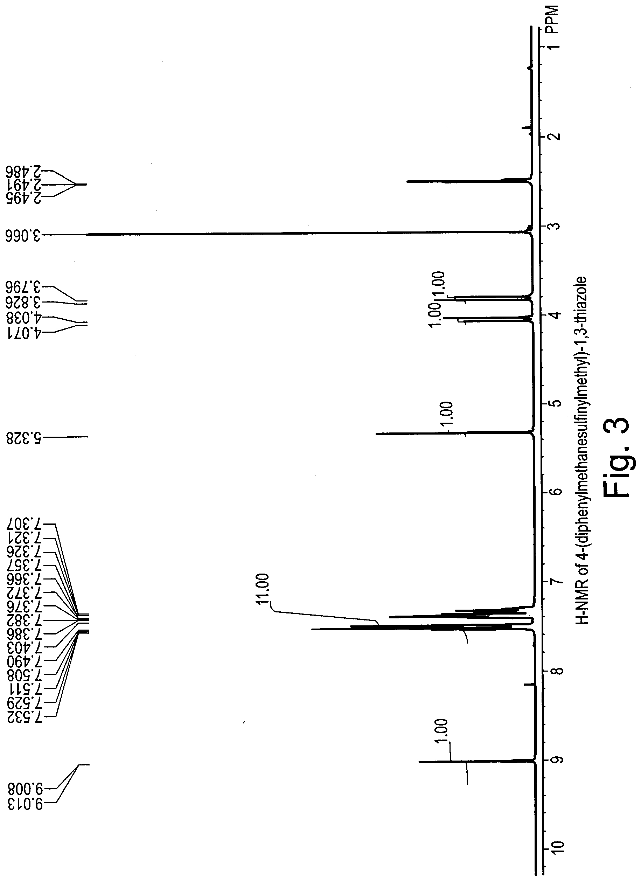

27.6 % H₂O₂ (0.74 mL) was added to the solution of thiazole 5 (0.0067 mol, 2.0 g) in glasial acetic acid (20 mL). The reaction mixture was stirred at room temperature during 24 h. The solvent was evaporated in vacuum till dryness. The residue was diluted with water, the precipitate was filtered off and purified by flash chromatography (hexane:CH₂Cl₂ 1:1) to yield 4-(diphenylmethanesulfinylmethyl)-1,3-thiazole, 1.87g as white solid, yield 89%. The H-NMR of 4-(diphenylmethanesulfinylmethyl)-1,3-thiazole (C2)is shown in Figure 3.

### 2. Synthesis of 2-(diphenylmethanesulfinylmethyl}-1,3-thiazole (C1):

### 2-(Benzhydrylthio)acetic acid

Thioglycolic acid (10.5 g, 7.92 ml (d=1.325), 0.114 mol) was added dropwise to the solution of diphenylmethanol in trifluoroacetic acid (100 ml). The formation of precipitate was observed in 30 min. The reaction mixture was stirred at room temperature for 3 h. The resulting precipitate was filtered off, washed with water (3×50 ml) and hexanes. The crude product was recrystallized from EtOAc/hexanes to get colorless precipitate of 2-(Benzhydrylthio)acetic acid. Yield: 17.93 g (64%). M.p. 121 °C.

### 2-(Benzhydrylthio)acetamide

2-(Benzhydrylthio)acetic acid (12.05 g, 0.0466 mol) was suspended in anhydrous benzene (60 ml) and the solution of SOCl₂ (22.197 g, 13.41 ml (d=1.655), 0.186 mol) in benzene (14 ml) was added to the suspension slowly. The mixture was refluxed for 2 h. Then the solvent was evaporated and the resulting oil was re-disolved in DCM, cooled in an ice-bath and dry NH₃ (gas) was bubbled in to the solution during 2 h. The resulting suspension was diluted by NaHCO₃ (10% aq. solution) and extracted with DCM. The organic layer was washed with brine and dried over Na₂SO₄. Solvent was evaporated and the formed colorless precipitate of 2-(Benzhydrylthio)acetamide was washed with ether. Yield: 69% (8.25 g). M.p. 95-100 °C.

### 2-(Benzhydrylthio)ethanethioamide

The mixture of amide 2-(Benzhydrylthio)acetamide (4 g, 0.0155 mol) and Lawesson's reagent (3.44 g, 0.0085 mol, 0.55 eq.) in anhydrous dioxane (50 ml) was refluxed for 2 h. The solvent was evaporated till dryness. The resulting yellow oil was dissolved in DCM, washed with water and brine. The solution was dried over Na₂SO₄. The crude product was purified with flash column chromatography (EtOAc/hexanes 1:2 → 1:1). Pure 2-(Benzhydrylthio)ethanethioamide was obtained as colorless precipitate (62%, 2.63 g). M.p. 96-99°C.

### 2-(Benzhydrylthiomethyl)thiazole

The solution of 2-(Benzhydrylthio)ethanethioamide (1.0g, mol, 1 eq.) and 1,2-dichloro-1-ethoxyethane (0.43 g, 0.37 mL, 0.003 mol, 2 eq.) in 10 mL of anhydrous dimethylformamide (DMF) was heated for 2 h at 90-95 °C and 2 h more at 120-125°C. The solvent was evaporated in vacuum till dryness. The black residue was dissolved in hot ethyl acetate and filtered through silica gel. The filtrate was concentrated in vacuum and the crude product was purified by flash column chromatography (silica gel, EtOAc/hexane = 1/6) to yield 2-(benzhydrylthiomethyl)thiazol in 34% as brownish precipitate. M.p. 72-74 °C.

### 2-(diphenylmethanesulfinylmethyl}-1,3-thiazole (C1)

To the solution of 2-(Benzhydrylthiomethyl)thiazole (0.39 g, 0.0013 mol) in glacial HOAc (4 ml) the solution of H₂O₂ (27.6%, d=1.105, 0.145 ml) was added. The reaction mixture was stirred during 24 h. The reaction mixture was poured on ice. The formed precipitate was filtered off. The crude product was purified by flash column chromatography (silica gel, EtOAc/hexane = 1/1) to yield **C1** in 74% as colorless precipitate. M.p. 119-122°C.

NMR (400 MHz, DMSO-d₆+CCl₄): 3.98 (1H, d, J = 14 Hz, CH₂), 4.28 (1H, d, J = 14 Hz, CH2), 5.38 (1H, s, CH), 7.31-7.42 (6H, m, H_{arom.}), 7.47 (2H, d, J = 7.2 Hz, Hₐᵣₒₘ.), 7.53 (2H, d, J = 7.2 Hz, H_{arom.}), 7.59 (1H, d, J = 3.6 Hz, H_{thiaz.}), 7.81 (1H, d, J = 3.6 Hz, CH_{thiaz.}).

MS: [M+1] = 314.06

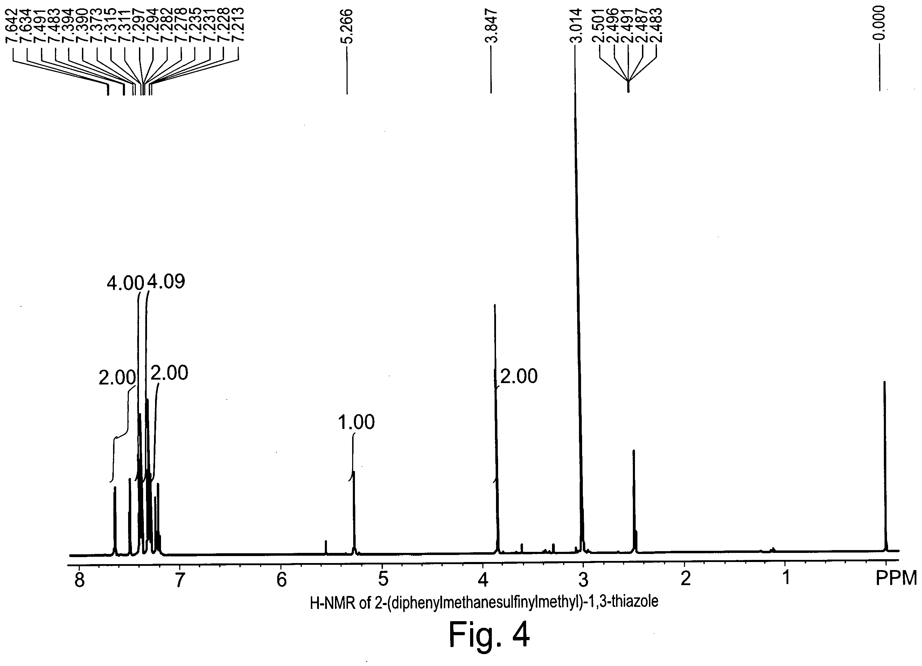

NMR of 2-(diphenylmethanesulfinylmethyl}-1,3-thiazole (C1) is shown in Figure 4.

### 3. Synthesis of 2-(diphenylmethanesulfinylmethyl)-4-methyl-1,3-thiazole (C3).

### 2-(Benzhydrylthiomethyl)-4-methylthiazole

α-Chloroacetone (0.41 ml, 0.00512 mol, 1.4 eq) was added to the solution of 2-(Benzhydrylthio)acetamide (1 g, 0.00366 mol, 1.0 eq.) in dry EtOH (16 ml) and the resulting mixture was refluxed during 7 h. The solvent was evaporated till dryness and small amount of EtOAc was added to the resulting oil. The oil was immediately transformed to colorless precipitate. The precipitate was filtered off and washed with EtOAc to obtained 4-methylthiazole in 76% yield (0.866 g). M.p. 154 °C.

### 2-(diphenylmethanesulfinylmethyl)-4-methyl-1,3-thiazole (C3)

To the solution of 4-methylthiazole (0.86 g, 0.00276 mol) in glacial HOAc (16 ml) the solution of H₂O₂ (27.6%, d=1.105, 0.31 ml) was added. The reaction mixture was stirred at 30-35 °C during 5 h. The reaction mixture was poured on ice, the resulting oil was extracted with EtOAc, washed with water. The organic layer was dried, evaporated till dryness. The resulting oil was coat with ether and the colorless precipitate was formed at low tempera-temperature. Crud product was recrystallized from EtOAc. Yield 61% (0.553 g), m.p. 104-107 °C.

NMR ¹H (CDCl₃, 400 MHz): 3.48 (s, 3H, CH₃), 3.97 (d, 1H, J=13.6 Hz, CH₂), 4.18 (d, 1H, J=13.6 Hz, CH₂), 5.13 (s, 1H, CH), (6.89 (s, 1H, CH_{thiazol.}), 7.32-7.42 (m, 6H, H_{arom.}), 7.48-7.50 (m, 4H, H_{arom.}).

MS: [M+1] = 328.08

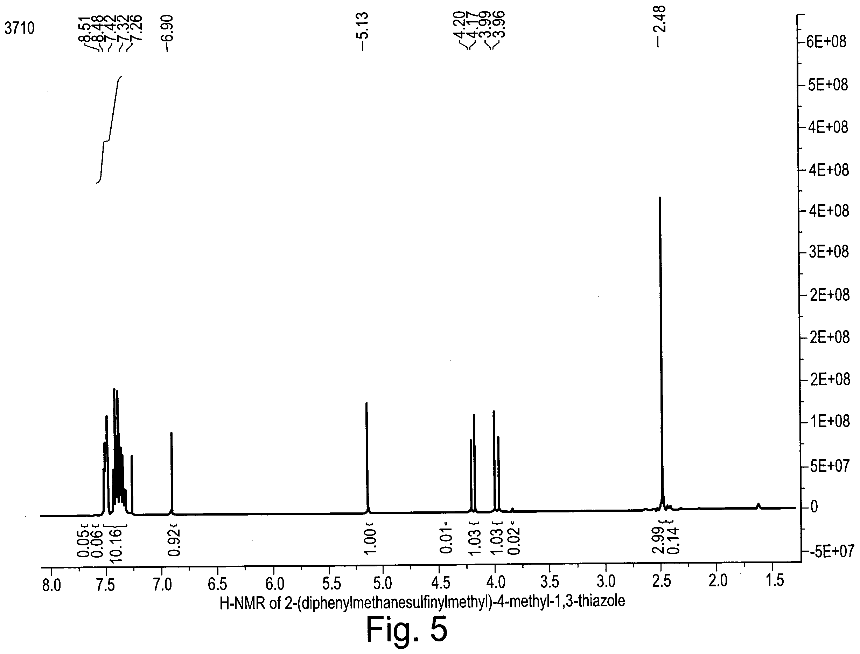

The NMR of 2-(diphenylmethanesulfinylmethyl)-4-methyl-1,3-thiazole (C3) is shown in Figure 5.

### 4. Synthesis of 4-(Diphenyl-methanesulfinylmethyl)-2-methyl-1,3-thiazole

### 4-Chloromethyl-2-methyl-thiazole hydrochloride

4-Chloromethyl-2-methyl-thiazole hydrochloride was obtained from Acros Organics (New Jersey, USA; Geel, Belgium). The compound can also be prepared according to literature procedures (Journal of Organic Chemistry, 1979, Volume 44, Issue 4, p. 497-501).

### [(Diphenylmethyl)sulfanyl]methanimideamide

Diphenylmethanol (13 g, 0.07 mol) and thiourea (6.5 g, 0.085 mol) are added in 0.5 L flask and 32.5 ml of water was added. The mixture was then heated to 95°C (an emulsion was obtained) and 26 g of 48% HBr (0.322 mol, 4.6 equivalents) was then gradually added during 0.5 h. The mixture was heated under reflux (106 - 107 °C) for 0.5 h and cooled to 80-85°C. The mixture was then cooled in ice and precipitate with crystals is formed. After filtration and washing with water colorless crystalline substance is obtained. The product was then dried in the high vacuum. 9.62 g of the product was obtained as a white crystalline solid (yield: 74%).

### 4-Benzhydrylsylfanylmethyl-2-methyl-thiazole

In a round bottom flask 3.51 g (10.86 mmol) of [(diphenylmethyl)sulfanyl]methanimideamide was dissolved in 75 ml of methanol. Afterwards, 2 g (10.86 mmol) of 4-chloromethyl-2-methyl-1,3-thiazole and 7.5 g (5 equivalents) of potassium carbonate were added to the mixture. The mixture was left to stir overnight at room temperature.

Methanol was evaporated and water was added. Then solution was extracted (3x) with 100 ml of ethylacetate. Organic phases were collected, combined, dried with Na₂SO4, filtered. Ethylacetate was removed by rotary evaporation. Semi-solid product was obtained. Crude product was purified via flash column chromatography on silica gel. 5% methanol in dichloromethane was used as mobile phase. 2.6 g of the crude solid product was obtained (yield: 59.07%).

NMR (200 MHz, DMSO-d₆): 2.63 (3H, s, CH₃), 3.62 (2H, s, CH₂), 5.33 (1H, s, CH), 7.15 (1H, s, CH_{thiazole}), 7.21-7.46 (10H, m, CH_{aromatic}).

Synthesis of 4-(Diphenyl-methanesulfinylmethyl)-2-methyl-1,3-thiazole

In a round bottom flask, 1.44 g (4.62 mmol) of 4-Benzhydrylsylfanylmethyl-2-methylthiazole was dissolved in 10 ml of glacial acetic acid (174.82 mmol). 0.55 ml (4.85 mmol) was dropped into the solution and stirred for 12 hrs.

Acid was neutralized with 5% sodium bicarbonate and ice. Aqueous mixture was extracted (3x) with 50 ml of ethyl acetate. Organic phases were collected, combined, dried with Na₂SO₄, filtered and ethyl acetate was concentrated on rotary evaporator.

Semi solid product was purified via flash column chromatography on silica gel. 5% methanol in dichloromethane was used as a mobile phase. Product was dried in a high vacuum. By this procedure 1.12 g of the final pure product was obtained (yield: 73.98%).

MS. [M+H⁺]=328.00 (MW is 327.46), [M+Na⁺]= 350.13.

NMR (200 MHz, DMSO-d₆): 2.65 (3H, s, CH₃), 3.73-3.99 (2H, dd, CH₂), 5.43 (1H, s, CH), 7.32-7.58 (11H, aromatic CH).

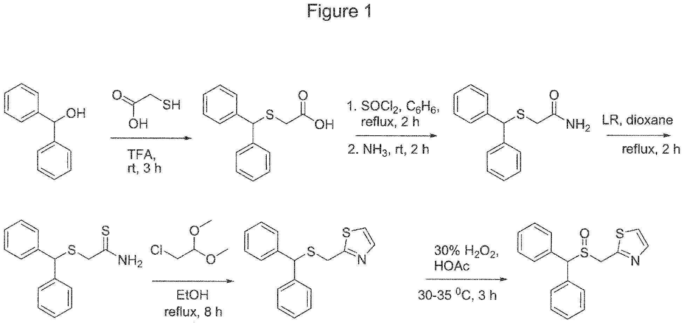

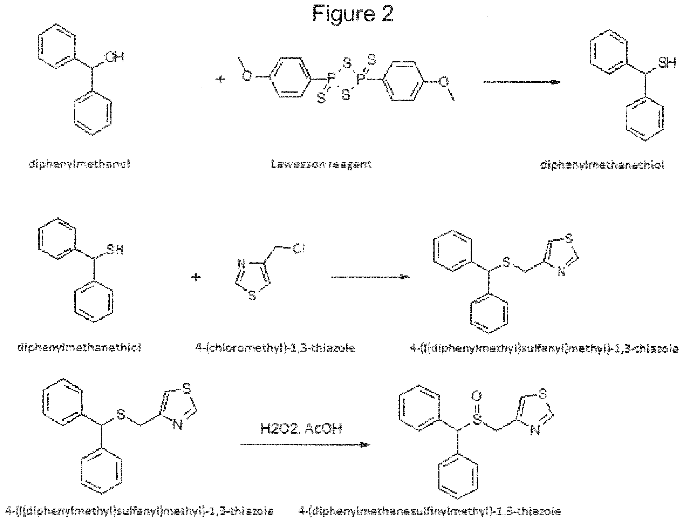

It is evident that the compounds according to the present invention can be provided by applying the above methods. For example, the compounds according to Fig. 5 can be prepared accordingly. In Figs. 1 and 2, such methods are exemplified by the two compounds produced above.

### B. Experiments demonstrating cognitive enhancing effects

The compound of the present invention can be used for cognitive enhancement of working memory of normal and aged individuals with and without a variety of human diseases, where inhibition of the improvement of working memory is beneficial.

The compounds of the present invention were demonstrated to have cognitive enhancing activity and more specifically working memory enhancing activity in an animal model. One such animal model is the radial arm maze using rats and is given in detail in Timofeva O. et al., Brain Res. 1308 (2010) 147-152: Wenk G. et al.; The Journal of Neuroscience (2004) 8.5A.1-8.5A.12; Akoto M. et al.; The Journal of Neuroscience (2000)20 (189: 7116-7121: Levin ED et al.; Behavioral Brain Res. 208 (2010) 319-327. Li-Bo Z. et al.; Neuropharmacology 37, 1998) 323-330. Roegge, CS. et al.; Toxicological Sciences 57, 121-30 (2000).

Male Sprague Dawley rats were used for the studies aged between 12-14 weeks. Twelve rats per group were used for this study. All rats were obtained from Core Unit of Biomedical Research, Division of Laboratory Animal Science and Genetics, Medical University of Vienna and maintained in cages made of makrolon and filled with autoclaved woodchips and water in bottles was available ad libitum. The room was illuminated with artificial light at an intensity of about 200 lx in 2 m from 5 am to 7 pm. Radial arm maze (RAM) training was performed between 8:00 h and 13:00 h.

All procedures were carried out according to the guidelines of the Ethics committee, Medical University of Vienna, and were approved by the Federal Ministry of Education, Science and Culture, Austria (BMWF-). All efforts were made to minimize animal suffering and to reduce the number of animals used.

### Radial arm maze:

Rats were trained in the 12 arm radial maze. Rats were handled for 5 days for adaptation (30 min/day/rat) and also to reduce the body weight to 85%. Water was provided ad libitum during the training. The amount of food was provided to maintain a lean, healthy body weight of approximately 85% of the free-feeding weight during the training.

The maze is made out of black plastic and kept at an elevation of 80 cm above the floor in a room with numerous visual cues. The central platform has a diameter of 50cm and 12 arms (12cm x 60cm) are projecting radially outwards. A plastic cylinder is used to restrict the movements of rats in the centre before being released to the start of training. The lifting of the cylinder is controlled by a pulley system from the far end of the room. Out of 12 arms, 8 arms were baited with food during the training and 4 arms remained un-baited. Food reward was placed in the baited arms 1cm from the distal end.

### Compound dosing

Before starting the training, rats were given two habituation sessions in which food was placed all over the maze and rats were allowed to explore the maze and consume the food for 5 minutes. During the training session, the same arms were baited for each rat once at the beginning of each session to assess working memory, while the other four arms were always left un-baited to test reference memory. The pattern of baited and un-baited arms was consistent throughout testing for each rat but differed among rats. Each trial began by placing the rat in the central platform, after 10 seconds the cylinder was lifted slowly and rats were allowed to enter any arm. The session lasted 8 minutes or until all 8 baited arms were entered. An arm was baited only once and a repeated entry into a baited arm was counted as a working memory error, whereas any entry into an un-baited arm was recorded as a reference memory error. The rats were given 10 training sessions over a period of 10 days.

The overall analysis of working and reference memory errors over the 10 sessions of acquisition was then carried out using ANOVA statistics

### C. Results

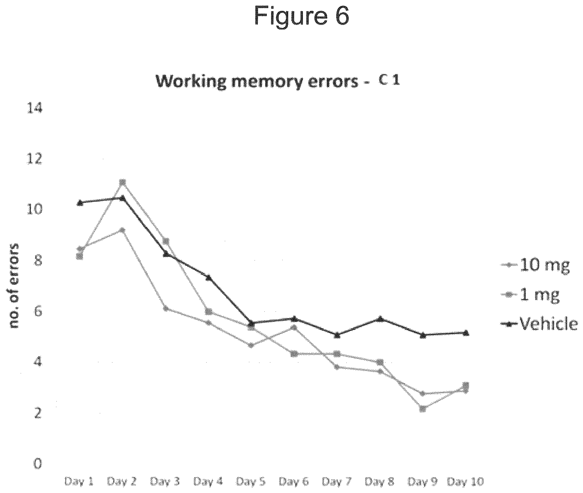

The results are depicted in Fig. 6. It can be seen that with the compound according to the present invention, a significant reduction in memory errors can be achieved, both, with the 1 mg and the 10 mg dose.

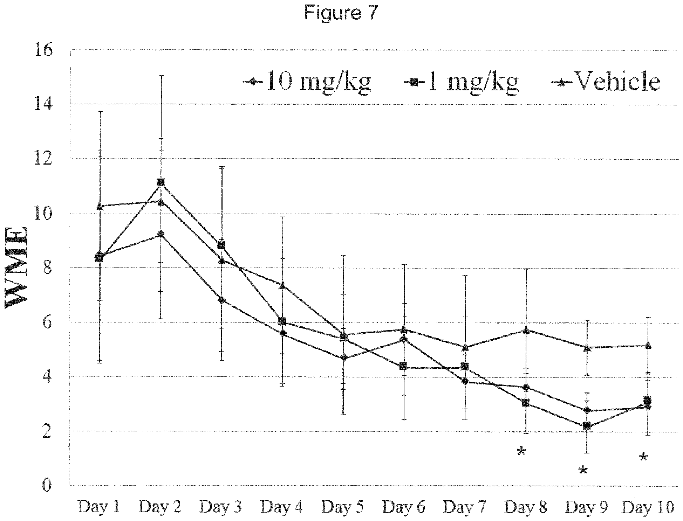

Rats that received C1 performed at a comparable level in the RAM training up to day seven, but from day eight TDI animals performed better as compared with their counterparts injected with DMSO. Data analysed used ANOVA and Bonferrini post hoc test. Trained rats showed decrease in working memory errors as days progressed (F (9, 750)=83.51; P < 0.0001). TDI rats continued to decrease working memory errors significantly from day eight to ten (F (6, 750)=7.630; P < 0.0001). ^{*} p < 0.05; *n* = 10-12 per group. Data is represented as mean ± SD in Fig. 7.

Although modifications and changes maybe suggested by those skilled in the art, it is the intention of the applicant to embody within the patent warranted hereon all changes and modifications as reasonably and probably come within the scope of this contribution to the art. The features of the present invention which are believed to be novel are set forth in detail in the appended claims. The features disclosed in the description, the figures as well as the claims could be essential alone or in every combination for the realization of the invention in its different embodiments.

## Claims

1. Chemical compound having the general formula (I): 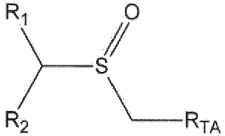 wherein R₁ and R₂ are, equal or independently, aryl, heteroaryl, fused aryl, fused heteroaryl, mono or multi-substituted aryl, mono or multi-substituted heteroaryl, mono or multi-substituted fused aryl, mono or multi-substituted fused heteroaryl; R_{TA} is a 2-1,3-, or 4-1,3- or 5-1,3-thiazole ring with the general Formula (IIa) or a 1,3,4-thiadiazole ring with the general Formula (IIb) or a 1,3,4-oxadiazole ring with the general Formula (IIc), 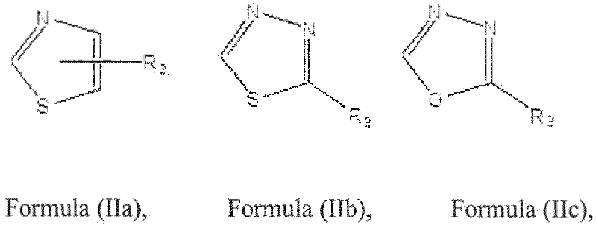 R₃ is present on the ring according to Formula (IIa), (IIb) or (IIc) 1 or 2 times, equal or independently,
wherein R₃ is selected from the group consisting of hydrogen, alkyl, cycloalkyl, beterocycloalkyl, alkylamino, in particular dialkylamino, arylamino, in particular diarylamino, hydroxyalkylamino, alkoxy, arylalkyl, hydroxyalkyl, thioalkyl, haloalkyl, haloaryl, haloarylalkyl, haloalkoxy, mono or multi-substituted alkyl, mono or multi-substituted cycloalkyl, mono or multi-substituted heterocycloalkyl, mono or multi-substituted alkylamino, in particular dialkylamino, mono or multi-substituted arylamino, in particular diarylamino, mono or multi-substituted hydroxyalkylamino, mono or multi-substituted alkoxy, mono or multi-substituted arylalkyl, mono or multi-substituted hydroxyalkyl, mono or multi-substituted thioalkyl, mono or multi-substituted haloalkyl, mono or multi-substituted haloaryl, mono or multi-substituted haloarylalkyl, mono or multi-substituted haloalkoxy and carboxylate ester,
wherein substituted means substituted with a residue selected from the group consisting of alkyl, cykloalkyl, heterocycloalkyl, hydroxyalkyl, alkylthio, ether, hydroxyl, fluoride, chloride, bromide and iodide.

2. Compound according to claim 1, wherein at least one of, in particular both of, R₁ and R₂ is/are aryl or substituted aryl, preferably phenyl or substituted phenyl.

3. Compound according to claim 1 or claim 2, wherein R_{TA} is a thiazole group, preferably a 2-thiazole group, in particular 2-thiazole, or a 4-thiazole group, in particular 2-methyl-4-thiazole, or wherein R_{TA} is selected from the group consisting of a thiadiazole group, preferably a 1,3,4-thiadiazole-5-oxyalkyl, and a 1,3,4-oxadiazole group, preferably 1,3,4-oxadiazole.

4. Compound according to any of claims 1 to 3, wherein alkyl is selected from the group consisting of methyl, ethyl, n-propyl, iso-propyl and tert-butyl, and/or wherein hydroxyalkyl is hydroxymethyl.

5. Compound according to any one of claims 1 to 4, wherein R3 is alkyl substituted with at least one residue selected from the group consisting of heterocycloalkyl, carboxylic acid, amide and ester, in particular methyl substituted with heterocycloalkyl, preferably heterocycloalkyl containing at least two heteroatoms.

6. Compound according to any one of claims 1 to 4, wherein R3 is hydrogen or alkyl, in particular methyl, or oxyalkyl, in particular oxymethyl.

7. Compound according to any one of claims 1 to 6, wherein it is selected from the group consisting of 2-(diphenylmethanesulfinylimethyl)-1,3-thiazole, 4-(diphenylmethanesulfinylmethyl)-1,3-thiazole, 2-(diphenylmethanesulfinylmethyl)-4-methyl-1,3-thiazole, 4-(diphenylmethanesulfinylmethyl)-2-methyl-1,3-thiazole, 2-(diphenylmethanesulfinylmethyl)-4-oxymethyl-1,3,4-thiadiazole, and 2-(diphenylmethanesulfinylmethyl)-1,3,4-oxadiazole, preferably 4-(diphenylmethanesulfinylmethyl)-2-methyl-1,3-thiazole.

8. Compound according to any one of claims 1 to 7 for use in therapy.

9. Compound according to any one of claims 1 to 7 for use in improving cognitive functions in human individuals, in particular in aging individuals.

10. Compound for use according to claim 9 wherein it is used in improving the short term memory and/or the working memory in human individuals, in particular for use in improving the working memory.

11. Compound for use according to claim 9 or 10, wherein the human individual does not have defects of cognitive functions and/or short term memory deficits and/or working memory deficits.

12. Compound for use according to claim 11, wherein the human individual does not have defects of cognitive functions and/or short term memory deficits and/or working memory deficits caused by diseases of the brain.

13. Compound for use according to claim 9 or 10, wherein the human individual has defects of cognitive functions and/or short term memory deficits and/or working memory deficits caused by diseases of the brain.

14. Compound for use according to claim 13, wherein the human individual has defects of cognitive functions and/or short term memory deficits and/or working memory deficits caused by Alzheimer; Down syndrome; vascular cognitive impairment; stroke; frontotemporal dementia; behavioural, semantic or progressive aphasia type dementia; dementia with Lewy bodies; subcortical dementias; Parkinson's disease dementia; alcohol related dementia; dementia caused by traumatic brain injury; Huntington's disease related dementia; AIDS-related dementia; attention deficit disorders; working memory deficiencies related to ageing; working memory disorders related to viral infections of the brain; or schizophrenia.

15. Compound for use according to any one of claims 8 to 14, wherein the compound is delivered in single or multiple dosage unit form, especially selected from the group consisting of tablets; caplets; capsules; cachets; troches; lozenges; dispersions; suppositories; ointments; cataplasms (poultices); pastes; powders; dressings; creams; plasters; solutions; patches; aerosols; gels; liquid dosage forms suitable for oral or mucosal administration to a patient, in particular aqueous or non-aqueous liquid suspensions, oil-in-water emulsions, water-in-oil liquid emulsions, solutions, or elixirs; liquid dosage forms suitable for parenteral administration to a patient; and sterile solids that can be reconstituted to provide liquid dosage forms suitable for parenteral administration to a patient, more preferably in the form of a capsule or a tablet.

16. Compound for use according to claim 15, wherein the capsule is a gelatine capsule comprising one or more emulsifying agents and wherein the compound is dissolved in at least one oil in said capsule.

17. Compound for use according to any one of claims 8 to 16, wherein the compound is administered in an amount of about 1 mg/kg to about 10 mg/kg body weight and/or is administered in an amount of about 0.1 mg to about 10 g, preferably of about 1 mg to about 1 g, and more preferably of about 10 mg to about 200 mg.

18. Compound for use according to any one of claims 8 to 17, wherein the compound is administered alone or in combination with one or more compounds selected from the group consisting of rivastigmin, donepezil, galanthamin, memantine, clozapine, risperidone, olanzapine, aripiprazole, quitiapine, D1 agonists, nicotinic alpha 7 agonists, d-serine, d-cycloserine, PDE2,4,9 inhibitors, AMPA agonists, lamotrigine, N-desmethylclozapine, mGlu receptor agonists, GABA A receptor agonists, and muscarinic 1 and 4 agonists.

19. Compound according to any one of claims 1 to 7 for use in improving cognitive functions in human individuals, who do not have defects of cognitive functions and/or short term memory deficits and/or working memory deficits, in particular for improving cognitive functions in human individuals, who do not have working memory deficits.

20. Compound for use according to claim 19, wherein the cognitive function to be improved is the short term memory and/or the working memory, in particular the working memory.

21. Pharmaceutical preparation comprising at least one compound according to any one of claims 1 to 7 and a pharmaceutically acceptable carrier and/or diluent.

22. Pharmaceutical preparation according to claim 21, wherein the preparation further comprises one or more compounds selected from the group consisting of rivastigmin, donepezil, galanthamin, memantine, clozapine, risperidone, olanzapine, aripiprazole, quitiapine, D1 agonists, nicotinic alpha 7 agonists, d-serine, d-cycloserine, PDE2,4,9 inhibitors, AMPA agonists, lamotrigine, N-desmethylelozapine, mGlu receptor agonists, GABA A receptor agonists, and muscarinic 1 and 4 agonists.

23. Pharmaceutical preparation according to claim 21 or claim 22, wherein the preparation is a single or multiple dosage unit form, in particular a single dosage unit form, selected from the group consisting of tablets; caplets; capsules; cachets; troches; lozenges; dispersions; suppositories; ointments; cataplasms (poultices); pastes; powders; dressings; creams; plasters; solutions; patches; aerosols; gels; liquid dosage forms suitable for oral or mucosal administration to a patient, in particular aqueous or non-aqueous liquid suspensions, oil-in-water emulsions, water-in-oil liquid emulsions, solutions, or elixirs; liquid dosage forms suitable for parenteral administration to a patient; and sterile solids that can be reconstituted to provide liquid dosage forms suitable for parenteral administration to a patient; more preferably in the form of a capsule or a tablet.

24. Pharmaceutical preparation according to claim 23, wherein the capsule is a gelatine capsule comprising one or more emulsifying agents and wherein the compound is dissolved in at least one oil in said capsule.

25. Pharmaceutical preparation according to any one of claims 21 to 24, wherein the preparation contains an amount of about 1 mg/kg to about 10 mg/kg body weight of the compound according to any one of claims 1 to 8 and/or wherein the preparation contains an amount of about 0.1 mg to about 10 g, preferably of about 1 mg to about 1 g, and more preferably of about 10 mg to about 200 mg, of the compound according to any one of claims 1 to 7.

26. Pharmaceutical preparation according to any one of claims 21 to 25 for use in therapy.

27. Pharmaceutical preparation according to any one of claims 21 to 25 for use in improving cognitive functions in human individuals, in particular for use in improving the short term memory and/or the working memory in human individuals, in particular for use in improving the working memory, in particular in aging individuals.

28. Process for the preparation of a compound according to any one of claims 1 to 7 comprising
reacting diarylmethanol, in particular benzhydrol, with Lawesson's reagent, treating the reaction product diarylmethanethiol with a cloromethyl-thiazole, -thiadiazole or - oxadiazole derivative, in particular with 2-(chlorarnethyl)-1,3-thiazole, 4-(chloromethyl)-1,3-thiazole, 2-(chloromethyl)-4-methyl-1,3-thiazole, 4-(chloromethyl)-2-methyl-1,3-thiazole, 2-(chloromethyl)-4-oxymethyl-1,3,4-thiadiazole, or 2-(chloromethyl)-1,3,4-oxadiazole, and oxidizing the ((diarylmethyl)sulfanyl)methyl-thiazole, -thiadiazole or -oxadiazole derivative to the corresponding diarylmethanesulfinylmethyl compound or comprising
reacting diarylmethanol, in particular benzhydrol, with thioglycolic acid to form 2-(diarylmehtylthio) acetic acid, converting said acetic acid derivative to 2-(diarylmethylthio) acetamide, converting said acetamide derivative, in particular by treatment with Lawesson's reagent, to the corresponding ethanethioamide derivative, converting said ethanethioamide derivative to a (((diarylmethy))sulfanyl)methyl) thiazole derivative, in particular to 2-(diphenylmethanesulfanylmethyl)-1,3-thiazole, 4-(diphenylmethanesulfanylmethyl)-1,3-thiazole, 2-(diphenylmethanesulfanylmethyl-4-methyl-1,3-thiazole or 4-(diphenylmethanesulfanylmethyl)-2-methyl-1,3-thiazole, and oxidizing said thiazole compound to the corresponding (diarylmethanesulfinylmethyl) thiazole derivative.

## Patentansprüche

1. Chemische Verbindung mit der allgemeinen Formel (I): 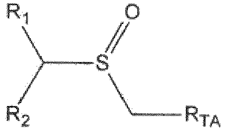 wobei R₁ und R₂, gleich oder unabhängig voneinander, Aryl, Heteroaryl, kondensiertes Aryl, kondensiertes Heteroaryl, ein- oder mehrfach substituiertes Aryl, ein- oder mehrfach substituiertes Heteroaryl, ein- oder mehrfach substituiertes kondensiertes Aryl, ein- oder mehrfach substituiertes kondensiertes Heteroaryl sind;
R_{TA} ein 2- oder 4- oder 5-substituierter 1,3-Thiazolring mit der allgemeinen Formel (IIa) oder ein 1,3,4-Thiadiazolring mit der allgemeinen Formel (IIb) oder ein 1,3,4-Oxadiazolring mit der allgemeinen Formel (IIc) ist, 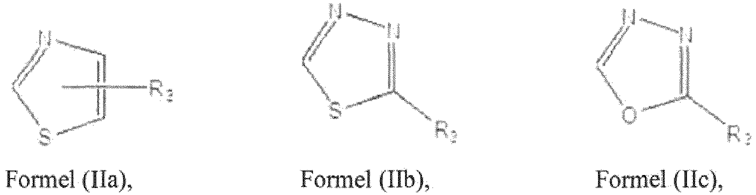 R₃ an dem Ring gemäß Formel (IIa), (IIb) oder (IIc) ein- oder zweimal, gleich oder unabhängig voneinander, vorliegt,
wobei R₃ ausgewählt ist aus der Gruppe bestehend aus Wasserstoff, Alkyl, Cycloalkyl, Heterocycloalkyl, Alkylamino, insbesondere Dialkylamino, Arylamino, insbesondere Diarylamino, Hydroxyalkylamino, Alkoxy, Arylalkyl, Hydroxyalkyl, Thioalkyl, Halogenalkyl, Halogenaryl, Halogenarylalkyl, Halogenalkoxy, ein- oder mehrfach substituiertem Alkyl, ein- oder mehrfach substituiertem Cycloalkyl, ein- oder mehrfach substituiertem Heterocycloalkyl, ein- oder mehrfach substituiertem
Alkylamino, insbesondere Dialkylamino, ein- oder mehrfach substituiertem Arylamino, insbesondere Diarylamino, ein- oder mehrfach substituiertem Hydroxyalkylamino, ein- oder mehrfach substituiertem Alkoxy, ein- oder mehrfach substituiertem Arylalkyl, ein- oder mehrfach substituiertem Hydroxyalkyl, ein- oder mehrfach substituiertem Thioalkyl, ein- oder mehrfach substituiertem Halogenalkyl, ein- oder mehrfach substituiertem Halogenaryl, ein- oder mehrfach substituiertem Halogenarylalkyl, ein- oder mehrfach substituiertem Halogenalkoxy und Carboxylatester,
wobei "substituiert" bedeutet: substituiert mit einem Rest, ausgewählt aus der Gruppe bestehend aus Alkyl, Cycloalkyl, Heterocycloalkyl, Hydroxyalkyl, Alkylthio, Ether, Hydroxyl, Fluorid, Chlorid, Bromid und Iodid.

2. Verbindung nach Anspruch 1, dadurch gegennzeichnet, dass mindestens eines von, insbesondere beide von, R₁ und R₂ Aryl oder substituiertes Aryl, vorzugsweise Phenyl oder substituiertes Phenyl, ist/sind.

3. Verbindung nach Anspruch 1 oder Anspruch 2, dadurch gegennzeichnet, dass R_{TA} ein Thiazolrest, vorzugsweise ein 2-substituierter Thiazolrest, insbesondere 2-substituiertes Thiazol, oder ein 4-substituierter Thiazolrest, insbesondere 4-substituiertes 2-Methylthiazol, ist oder wobei R_{TA} ausgewählt ist aus der Gruppe bestehend aus einem Thiadiazolrest, vorzugsweise einem 5-Oxyalkyl-1,3,4-thiadiazol, und einem 1,3,4-Oxadiazolrest, vorzugsweise 1,3,4-Oxadiazol.

4. Verbindung nach einem der Ansprüche 1 bis 3, dadurch gegennzeichnet, dass das Alkyl ausgewählt ist aus der Gruppe bestehend aus Methyl, Ethyl, n-Propyl, Isopropyl und tert-Butyl, und/oder wobei das Hydroxyalkyl Hydroxymethyl ist.

5. Verbindung nach einem der Ansprüche 1 bis 4, dadurch gegennzeichnet, dass R₃ Alkyl, substituiert mit mindestens einem Rest, ausgewählt aus der Gruppe bestehend aus Heterocycloalkyl, Carbonsäure, Amid und Ester, insbesondere Methyl substituiert mit Heterocycloalkyl, vorzugsweise Heterocycloalkyl, das mindestens zwei Heteroatome enthält, ist.

6. Verbindung nach einem der Ansprüche 1 bis 4, dadurch gegennzeichnet, dass R₃ Wasserstoff oder Alkyl, insbesondere Methyl, oder Oxyalkyl, insbesondere Oxymethyl, ist.

7. Verbindung nach einem der Ansprüche 1 bis 6, dadurch gegennzeichnet, dass diese ausgewählt ist aus der Gruppe bestehend aus 2-(Diphenylmethansulfinyimethyl)-1,3-thiazol, 4-(Diphenylmethansulfinylmethyl)-1,3-thiazol, 2-(Diphenylmethansulfinylmethyl)-4-methyl-1,3-thiazol, 4-(Diphenylmethansulfinylmethyl)-2-methyl-1,3-thiazol, 2-(Diphenylmethansulfinylmethyl)-4-oxymethyl-1,3,4-thiadiazol und 2-(Diphenylmethansulfinylmethyl)-1,3,4-oxadiazol, vorzugsweise 4-(Diphenylmethansulfinylmethyl)-2-methyl-1,3-thiazol.

8. Verbindung nach einem der Ansprüche 1 bis 7 zur Verwendung in der Therapie.

9. Verbindung nach einem der Ansprüche 1 bis 7 zur Verwendung bei der Verbesserung kognitiver Funktionen bei menschlichen Individuen, insbesondere bei alternden Individuen.

10. Verbindung zur Verwendung nach Anspruch 9, dadurch gegennzeichnet, dass sie bei der Verbesserung des Kurzzeitgedächtnisses und/oder des Arbeitsgedächtnisses bei menschlichen Individuen, insbesondere zur Verwendung bei der Verbesserung des Arbeitsgedächtnisses, verwendet wird.

11. Verbindung zur Verwendung nach Anspruch 9 oder 10, dadurch gegennzeichnet, dass das menschliche Individuum keine Störungen kognitiver Funktionen und/oder Defizite des Kurzzeitgedächtnisses und/oder Defizite des Arbeitsgedächtnisses aufweist.

12. Verbindung zur Verwendung nach Anspruch 11, dadurch gegennzeichnet, dass das menschliche Individuum keine durch Erkrankungen des Gehirns verursachten Störungen kognitiver Funktionen und/oder Defizite des Kurzzeitgedächtnisses und/oder Defizite des Arbeitsgedächtnisses aufweist.

13. Verbindung zur Verwendung nach Anspruch 9 oder 10, dadurch gegennzeichnet, dass das menschliche Individuum durch Erkrankungen des Gehirns verursachte Störungen kognitive Funktionen und/oder Defizite des Kurzzeitgedächtnisses und/oder Defizite des Arbeitsgedächtnisses aufweist.

14. Verbindung zur Verwendung nach Anspruch 13, dadurch gegennzeichnet, dass das menschliche Individuum Störungen kognitiver Funktionen und/oder Defizite des Kurzzeitgedächtnisses und/oder Defizite des Arbeitsgedächtnisses aufweist, die verursacht sind durch Alzheimer; Down-Syndrom; vaskuläre kognitive Störung; Schlaganfall; frontotemporale Demenz; Demenz vom Verhaltenstyp der semantischen oder progressiven Aphasie; Lewy-Body-Demenz; subkortikale Demenzen; Demenz bei Parkinson-Krankheit; alkoholbedingte Demenz; durch traumatische Hirnschädigung verursachte Demenz; durch Chorea Huntington bedingte Demenz; durch AIDS bedingte Demenz; Aufmerksamkeitsdefizitstörungen; durch Alterung bedingte Defizite des Arbeitsgedächtnisses; durch Virusinfektionen des Gehirn bedingte Störungen des Arbeitsgedächtnisses; oder Schizophrenie.

15. Verbindung zur Verwendung nach einem der Ansprüche 8 bis 14, dadurch gegennzeichnet, dass
die Verbindung in Einzel- oder Mehrfachdosierungsform abgegeben wird, insbesondere ausgewählt aus der Gruppe bestehend aus Tabletten; Capletten; Kapseln; Oblatenkapseln; Pastillen; Lutschtabletten; Dispersionen; Zäpfchen; Salben; Kataplasmen (Breiumschlägen); Pasten; Pudern; Verbandsmaterialien; Cremes; Pflastern; Lösungen; Patches; Aerosolen; Gelen; flüssigen Darreichungsformen, die für die orale oder mukosale Verabreichung an einen Patienten geeignet sind, insbesondere wässrigen oder nichtwässrigen flüssigen Suspensionen, Öl-in-Wasser-Emulsionen, flüssigen Wasser-in-Öl-Emulsionen, Lösungen oder Elixieren; flüssigen Darreichungsformen, die für die parenterale Verabreichung an einem Patienten geeignet sind; und sterilen Feststoffen, die rekonstituiert werden können, um flüssige Darreichungsformen bereitzustellen, die für die parenterale Verabreichung an einen Patienten geeignet sind, besonders bevorzugt in Form einer Kapsel oder einer Tablette.

16. Verbindung zur Verwendung nach Anspruch 15, dadurch gegennzeichnet, dass die Kapsel eine Gelatinekapsel, umfassend einen oder mehrere Emulgatoren, ist und wobei die Verbindung in mindestens einem Öl in der Kapsel gelöst ist.

17. Verbindung zur Verwendung nach einem der Ansprüche 8 bis 16, dadurch gegennzeichnet, dass
die Verbindung in einer Menge von etwa 1 mg/kg bis etwa 10 mg/kg Körpergewicht verabreicht wird und/oder in einer Menge von etwa 0,1 mg bis etwa 10 g, vorzugsweise von etwa 1 mg bis etwa 1 g, und stärker bevorzugt von etwa 10 mg bis etwa 200 mg, verabreicht wird.

18. Verbindung zur Verwendung nach einem der Ansprüche 8 bis 17, dadurch gegennzeichnet, dass
die Verbindung allein oder in Kombination mit einer oder mehreren Verbindungen, ausgewählt aus der Gruppe bestehend aus Rivastigmin, Donepezil, Galantamin, Memantin, Clozapin, Risperidon, Olanzapin, Aripiprazol, Quitiapin, D1-Agonisten, α7-Nicotin-Agonisten, D-Serin, D-Cycloserin, PDE-2-Inhibitoren, PDE-4-Inhibitoren, PDE-9-Inhibitoren, AMPA-Agonisten, Lamotrigin, N-Desmethylclozapin, mGlu-Rezeptor-Agonisten, GABA-A-Rezeptor-Agonisten und Muscarin-Agonisten 1 und 4, verabreicht wird.

19. Verbindung nach einem der Ansprüche 1 bis 7 zur Verwendung bei der Verbesserung kognitiver Funktionen bei menschlichen Individuen, die keine Störungen kognitiver Funktionen und/oder Defizite des Kurzzeitgedächtnisses und/oder Defizite des Arbeitsgedächtnisses aufweisen, insbesondere zur Verbesserung kognitiver Funktionen bei menschlichen Individuen, die keine Defizite des Arbeitsgedächtnisses aufweisen.

20. Verbindung zur Verwendung nach Anspruch 19, dadurch gegennzeichnet, dass die zu verbessernde kognitive Funktion das Kurzzeitgedächtnis und/oder das Arbeitsgedächtnis, insbesondere das Arbeitsgedächtnis, ist.

21. Pharmazeutische Zubereitung, umfassend mindestens eine Verbindung nach einem der Ansprüche 1 bis 7 und einen pharmazeutisch akzeptablen Träger und/oder ein pharmazeutisch akzeptables Verdünnungsmittel.

22. Pharmazeutische Zubereitung nach Anspruch 21, dadurch gegennzeichnet, dass die Zubereitung weiterhin eine oder mehrere Verbindungen, ausgewählt aus der Gruppe bestehend aus Rivastigmin, Donepezil, Galantamin, Memantin, Clozapin, Risperidon, Olanzapin, Aripiprazol, Quitiapin, D1-Agonisten, α7-Nicotin-Agonisten, D-Serin, D-Cycloserin, PDE-2-Inhibitoren, PDE-4-Inhibitoren, PDE-9-Inhibitoren, AMPA-Agonisten, Lamotrigin, N-Desmethylclozapin, mGlu-Rezeptor-Agonisten, GABA-A-Rezeptor-Agonisten und Muscarin-Agonisten 1 und 4, umfasst.

23. Pharmazeutische Zubereitung nach Anspruch 21 oder Anspruch 22, dadurch gegennzeichnet, dass
die Zubereitung eine Einzel- oder Mehrfachdosierungsform ist, insbesondere eine Einzeldosierungsform, ausgewählt aus der Gruppe bestehend aus Tabletten; Capletten; Kapseln; Oblatenkapseln; Pastillen; Lutschtabletten; Dispersionen; Zäpfchen; Salben; Kataplasmen (Breiumschlägen); Pasten; Pudern; Verbandsmaterialien; Cremes; Pflastern; Lösungen; Patches; Aerosolen; Gelen; flüssigen Darreichungsformen, die für die orale oder mukosale Verabreichung an einen Patienten geeignet sind, insbesondere wässrigen oder nichtwässrigen flüssigen Suspensionen, Öl-in-Wasser-Emulsionen, flüssigen Wasser-in-Öl-Emulsionen, Lösungen oder Elixieren; flüssigen Darreichungsformen, die für die parenterale Verabreichung an einen Patienten geeignet sind; und sterilen Feststoffen, die rekonstituiert werden können, um flüssige Darreichungsformen bereitzustellen, die für die parenterale Verabreichung an einen Patienten geeignet sind, stärker bevorzugt in Form einer Kapsel oder einer Tablette.

24. Pharmazeutische Zubereitung nach Anspruch 23, dadurch gegennzeichnet, dass die Kapsel eine Gelatinekapsel, umfassend einen oder mehrere Emulgatoren, ist und wobei die Verbindung in mindestens einem Öl in der Kapsel gelöst ist.

25. Pharmazeutische Zubereitung nach einem der Ansprüche 21 bis 24, dadurch gegennzeichnet, dass
die Zubereitung eine Menge von etwa 1 mg/kg bis etwa 10 mg/kg Körpergewicht der Verbindung nach einem der Ansprüche 1 bis 8 enthält und/oder die Zubereitung eine Menge von etwa 0,1 mg bis etwa 10 g, vorzugsweise von etwa 1 mg bis etwa 1 g, und stärker bevorzugt von etwa 10 mg bis etwa 200 mg, der Verbindung nach einem der Ansprüche 1 bis 7 enthält.

26. Pharmazeutisch Zubereitung nach einem der Ansprüche 21 bis 25 zur Verwendung in der Therapie.

27. Pharmazeutische Zubereitung nach einem der Ansprüche 21 bis 25 zur Verwendung bei der Verbesserung kognitiver Funktionen bei menschlichen Individuen, insbesondere zur Verwendung bei der Verbesserung des Kurzzeitgedächtnisses und/oder des Arbeitsgedächtnisses bei menschlichen Individuen, insbesondere zur Verwendung bei der Verbesserung des Arbeitsgedächtnisses, insbesondere bei alternden Individuen.

28. Verfahren zur Herstellung einer Verbindung nach einem der Ansprüche 1 bis 7, umfassend
Umsetzen von Diarylmethanol, insbesondere Benzhydrol, mit Lawesson-Reagens, Behandeln des Reaktionsproduktes Diarylmethanthiol mit einem Chlormethylthiazol-, Chlormethylthiadiazol- oder Chlormethyloxadiazol-Derivat, insbesondere mit 2-(Chlormethyl)-1,3-thiazol, 4-(Chlormethyl)-1,3-thiazol, 2-(Chlonrmethyl)-4-methyl-1,3-thiazol, 4-(Chlormethyl)-2-methyl-1,3-thiazol, 2-(Chlormethyl)-4-oxymethyl-1,3,4-thiadiazol oder 2-(Chlormethyl)-1,3,4-oxadiazol, und Oxidieren des ((Diarylmethyl)sulfanyl)methyl-thiazol-, (Diarylmethyl)sulfanyl)methyl-thiadiazol- oder ((Diarylmethyl)sulfanyl)methyl-oxadiazol-Derivats zu der entsprechenden Diarylmethansulfinylmethyl-Verbindung,
oder umfassend
Umsetzen von Diarylmethanol, insbesondere Benzhydrol, mit Thioglycolsäure unter Bildung von 2-(Diarylmethylthio)essigsäure, Umwandeln des Essigsäure-Derivats zu 2-(Diarylmethylthio)acetamid, Umwandeln des Acetamid-Derivats, insbesondere durch Behandlung mit Lawesson-Reagens, zu dem entsprechenden Ethanthioamid-Derivat, Umwandeln des Ethanthioamid-Derivats zu einem
(((Diarylmethyl)sulfanyl)methyl)thiazol-Derivat, insbesondere zu 2-(Diphenylmethansulfanylmethyl)-1,3-thiazol, 4-(Diphenylmethansulfanylmethyl)-1,3-thiazol, 2-(Diphenylmethansulfanylmethyl)-4-methyl-1,3-thiazol oder 4-(Diphenylmethansulfanylmethyl)-2-methyl-1,3-thiazol, und Oxidieren der Thiazolverbindung zu dem entsprechenden (Diarylmethansulfinylmethyl)thiazol-Derivat.

## Revendications

1. Composé chimique de formule générale (I) suivante : 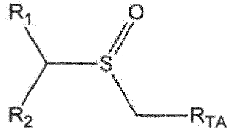 où R₁ et R₂ sont, de manière identique ou indépendante, un groupe aryle, hétéroaryle, aryle condensé, hétéroaryle condensé, aryle mono- ou multi-substitué, hétéroaryle mono- ou multi-substitué, aryle condensé mono- ou multi-substitué, hétéroaryle condensé mono- ou multi-substitué ;
R_{TA} est un cycle 2-1,3-, ou 4-1,3- ou 5-1,3-thiazole de formule générale (IIa) ou un cycle 1,3,4-thiadiazole de formule générale (IIb) ou un cycle 1,3,4-oxadiazole de formule générale (IIc), 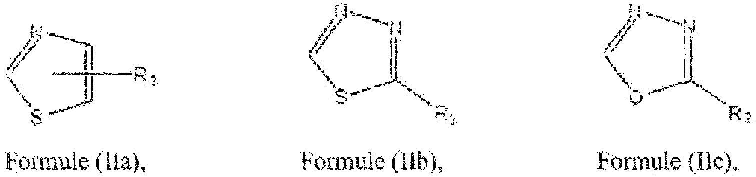 R₃ est présent 1 ou 2 fois sur le cycle correspondant à la formule (IIa), (IIb) ou (IIc), de manière identique ou indépendante,
R₃ étant sélectionné dans le groupe constitué d'hydrogène, alkyle, cycloalkyle, hétérocycloalkyle, alkylamino, en particulier dialkylamino, arylamino, en particulier diarylamino, hydroxyalkylamino, alcoxy, arylalkyle, hydroxyalkyle, thioalkyle, haloalkyle, halogénoaryle, halogénoarylalkyle, haloalcoxy mono- ou multi-substitué, alkyle mono- ou multi-substitué, cycloalkyle mono- ou multi-substitué, hétérocycloalkyle, alkylamino mono- ou multi-substitué, en particulier dialkylamino, arylamino mono- ou multi-substitué, en particulier diarylamino, hydroxyalkylamino mono- ou multi-substitué, alcoxy mono- ou multi-substitué, arylalkyle mono- ou multi-substitué, hydroxyalkyle mono- ou multi-substitué, thioalkyle mono- ou multi-substitué, haloalkyle mono- ou multi-substitué, halogénoaryle mono- ou multi-substitué, halogénoarylalkyle mono- ou multi-substitué, haloalcoxy et ester carboxylate mono- ou multi-substitués,
« substitué » signifiant substitué avec un résidu sélectionné dans le groupe constitué d'alkyle, cykloalkyle, hétérocycloalkyle, hydroxyalkyle, alkylthio, éther, hydroxyle, fluorure, chlorure, bromure et iodure.

2. Composé selon la revendication 1, où R₁ ou R₂, et en particulier R₁ et R₂ sont un aryle ou un aryle substitué, préférentiellement un phényle ou un phényle substitué.

3. Composé selon la revendication 1 ou la revendication 2, où R_{TA} est un groupe thiazole, préférentiellement un groupe 2-thiazole, en particulier 2-thiazole, ou un groupe 4-thiazole, en particulier 2-méthyle-4-thiazole, ou bien où R_{TA} est sélectionné dans le groupe constitué d'un groupe thiadiazole, préférentiellement 1,3,4-thiadiazole-5-oxyalkyle, et un groupe 1,3,4-oxadiazole, préférentiellement 1,3,4-oxadiazole.

4. Composé selon l'une des revendications 1 à 3, où l'alkyle est sélectionné dans le groupe constitué de méthyle, éthyle, n-propyle, iso-propyle et tert-butyle, et/ou où l'hydroxyalkyle est un hydroxyméthyle.

5. Composé selon l'une des revendications 1 à 4, où R₃ est un alkyle substitué par au moins un résidu sélectionné dans le groupe constitué d'hétérocycloalkyle, acide carboxylique, amide et ester, en particulier méthyle substitué par hétérocycloalkyle, préférentiellement hétérocycloalkyle contenant au moins deux hétéroatomes.

6. Composé selon l'une des revendications 1 à 4, où R₃ est l'hydrogène ou un alkyle, en particulier un méthyle, ou un oxyalkyle, en particulier un oxyméthyle.

7. Composé selon l'une des revendications 1 à 6, celui-ci étant sélectionné dans le groupe constitué de 2-(diphényle méthane sulfinyle méthyle)-1,3-thiazole, 4-(diphényle méthane sulfinyle méthyle)-1,3-thiazole, 2-(diphényle méthane sulfinyle méthyle)-4-methyl-1,3-thiazole, 4-(diphényle méthane sulfinyle méthyle)-2-méthyle-1,3-thiazole, 2-(diphényle méthane sulfinyle méthyle)-4-oxyméthyle-1,3,4-thiadiazole, et 2-(diphényle méthane sulfinyle méthyle)-1,3,4-oxadiazole, préférentiellement 4-(diphényle méthane sulfinyle méthyle)-2-méthyle-1,3-thiazole,

8. Composé selon l'une des revendications 1 à 7, destiné à une utilisation thérapeutique.

9. Composé selon l'une des revendications 1 à 7, destiné à améliorer les fonctions cognitives de sujets humains, en particulier de sujets vieillissants.

10. Composé destiné à une utilisation selon la revendication 9, où celui-ci est utilisé pour améliorer la mémoire à court terme et/ou la mémoire de travail de sujets humains, en particulier pour améliorer la mémoire de travail.

11. Composé destiné à une utilisation selon la revendication 9 ou la revendication 10, où le sujet humain ne présente pas de défaillance des fonctions cognitives et/ou de déficit de mémoire à court terme et/ou de mémoire de travail.

12. Composé destiné à une utilisation selon la revendication 11, où le sujet humain ne présente pas de défaillance des fonctions cognitives et/ou de déficit de mémoire à court terme et/ou de mémoire de travail dus à une affection cérébrale.

13. Composé destiné à une utilisation selon la revendication 9 ou la revendication 10, où le sujet humain présente une défaillance des fonctions cognitives et/ou un déficit de mémoire à court terme et/ou de mémoire de travail dus à une affection cérébrale.

14. Composé destiné à une utilisation selon la revendication 13, où le sujet humain présente une défaillance des fonctions cognitives et/ou un déficit de mémoire à court terme et/ou de mémoire de travail dus à la maladie d'Alzheimer ; au syndrome de Down ; à un déficit cognitif d'origine vasculaire ; à un accident vasculaire cérébral ; à la démence frontotemporale ; à une démence comportementale, sémantique ou de type à aphasie progressive ; à une démence à corps de Lewy ; à une démence sous-corticale ; à une démence liée à la maladie de Parkinson ; à une démence liée à l'alcool ; à une démence due à un traumatisme craniocérébral ; à une démence liée à la maladie de Huntington ; à une démence liée au SIDA ; à des troubles de déficit de l'attention ; à des défaillances de la mémoire de travail dues au vieillissement; à des troubles de la mémoire de travail dus à des infections virales du cerveau ; ou à la schizophrénie.

15. Composé destiné à une utilisation selon l'une des revendications 8 à 14, où ledit composé est administré sous une forme de dosage à une ou plusieurs unités, notamment sélectionnée dans le groupe constitué de gélules ; comprimés ; capsules ; cachets ; pastilles ; pilules ; dispersions ; suppositoires ; pommades ; cataplasmes (sinapismes) ; pâtes ; poudres ; pansements ; crèmes ; bandages ; solutions ; compresses ; aérosols ; gels ; formes de dosage liquide adaptées pour une administration orale ou par voie mucosale à un patient, en particulier suspensions liquides aqueuses ou non aqueuses, émulsions huile dans l'eau, émulsions liquides eau dans huile, solutions ou élixirs ; formes de dosage liquide adaptées pour une administration par voie parentérale à un patient ; et solides stériles pouvant être reconstitués pour obtenir des formes de dosage liquide adaptées pour une administration par voie parentérale à un patient, préférentiellement sous forme de capsule ou de comprimé.

16. Composé destiné à une utilisation selon la revendication 15, où la capsule est une capsule en gélatine comprenant un ou plusieurs agents émulsifiants et où le composé est dissous dans au moins une huile dans la capsule.

17. Composé destiné à une utilisation selon l'une des revendications 8 à 16, où le composé est administré dans une teneur comprise entre env. 1 mg/kg et env. 10 mg/kg de poids corporel et/ou est administré dans une teneur comprise entre env. 0,1 mg et env. 10 g, préférentiellement comprise entre env. 1 mg et env. 1 g, et plus particulièrement comprise entre env. 10 mg et env. 200 mg.

18. Composé destiné à une utilisation selon l'une des revendications 8 à 17, où le composé est administré seul ou en combinaison avec un ou plusieurs composés sélectionnés dans le groupe constitué de rivastigmine, donépézil, galantamine, mémantine, clozapine, rispéridone, olanzapine, aripiprazole, quétiapine, agonistes D1, agonistes du récepteur alpha 7 nicotinique, D-sérine, D-cyclosérine, inhibiteurs de la PDE2,4,9, agonistes AMPA, lamotrigine, N-desméthyl clozapine, agonistes du récepteur mGlu, agonistes du récepteur GABA A et agonistes des récepteurs muscariniques 1 et 4.

19. Composé selon l'une des revendications 1 à 7, destiné à améliorer les fonctions cognitives de sujets humains ne présentant pas de défaillance des fonctions cognitives et/ou de déficit de mémoire à court terme et/ou de mémoire de travail, en particulier à améliorer les fonctions cognitives de sujets humains ne présentant pas de déficit de mémoire de travail.

20. Composé destiné à une utilisation selon la revendication 19, où la fonction cognitive à améliorer est la mémoire à court terme et/ou la mémoire de travail, en particulier la mémoire de travail.

21. Préparation pharmaceutique comprenant au moins un composé selon l'une des revendications 1 à 7 et un excipient et/ou un diluent pharmaceutiquement acceptables.

22. Préparation pharmaceutique selon la revendication 21, où ladite préparation comprend en outre un ou plusieurs composés sélectionnés dans le groupe constitué de rivastigmine, donépézil, galantamine, mémantine, clozapine, rispéridone, olanzapine, aripiprazole, quétiapine, agonistes D1, agonistes du récepteur alpha 7 nicotinique, D-sérine, D-cyclosérine, inhibiteurs de la PDE2,4,9, agonistes AMPA, lamotrigine, N-desméthyl clozapine, agonistes du récepteur mGlu, agonistes du récepteur GABA A et agonistes des récepteurs muscariniques 1 et 4.

23. Préparation pharmaceutique selon la revendication 21 ou la revendication 22, où ladite préparation est une forme de dosage à une ou plusieurs unités, en particulier une forme de dosage à une unité, sélectionnée dans le groupe constitué de gélules ; comprimés ; capsules ; cachets ; pastilles ; pilules ; dispersions ; suppositoires ; pommades ; cataplasmes (sinapismes) ; pâtes ; poudres ; pansements ; crèmes ; bandage solutions ; compresses ; aérosols ; gels ; formes de dosage liquide adaptées pour une administration orale ou par voie mucosale à un patient, en particulier suspensions liquides aqueuses ou non aqueuses, émulsions huile dans l'eau, émulsions liquides eau dans huile, solutions ou élixirs ; formes de dosage liquide adaptées pour une administration par voie parentérale à un patient ; et solides stériles pouvant être reconstitués pour obtenir des formes de dosage liquide adaptées pour une administration par voie parentérale à un patient, préférentiellement sous forme de capsule ou de comprimé.

24. Préparation pharmaceutique selon la revendication 23, où la capsule est une capsule en gélatine comprenant un ou plusieurs agents émulsifiants et où le composé est dissous dans au moins une huile dans la capsule.

25. Préparation pharmaceutique selon l'une des revendications 21 à 24, où ladite préparation contient une teneur comprise entre env. 1 mg/kg et env. 10 mg/kg de poids corporel du composé selon l'une des revendications 1 à 8, et/ou où ladite préparation contient une teneur comprise entre env. 0,1 mg et env. 10 g, préférentiellement comprise entre env. 1 mg et env. 1 g, et plus particulièrement comprise entre env. 10 mg et env. 200 mg du composé selon l'une des revendications 1 à 7.

26. Préparation pharmaceutique selon l'une des revendications 21 à 25, destinée à une utilisation thérapeutique.

27. Préparation pharmaceutique selon l'une des revendications 21 à 25, destiné à améliorer les fonctions cognitives de sujets humains, en particulier destiné à améliorer la mémoire à court terme et/ou la mémoire de travail de sujets humains, en particulier pour améliorer la mémoire de travail, en particulier de sujets vieillissants.

28. Procédé de préparation d'un composé selon l'une des revendications 1 à 7 comprenant un diarylméthanol réactif, en particulier benzhydrol, avec réactif de Lawesson, traitement du produit de réaction diarylméthanethiol avec un dérivé chlorométhyle-thiazole, -thiadiazole ou - oxadiazole, en particulier avec 2-(chlorométhyle)-1,3-thiazole, 4-(chlorométhyle)-1,3-thiazole, 2-(chlorométhyle)-4-méthyle-1,3-thiazole, 4-(chlorométhyle)-2-méthyle-1,3-thiazole, 2-(chlorométhyle)-4-oxyméthyle-1,3,4-thiadiazole, ou 2-(chlorométhyle)-1,3,4-oxadiazole, et oxydation du dérivé ((diarylméthyle)sulfanyle)méthyle-thiazole, -thiadiazole ou -oxadiazole en composé diarylméthanesulfinylméthyle correspondant, ou comprenant
un diarylméthanol réactif, en particulier benzhydrol, avec acide thioglycolique pour former un acide acétique 2-(diarylméthylthio), conversion dudit dérivé d'acide acétique en 2-(diarylméthylthio) acétamide, conversion dudit dérivé d'acétamide, en particulier par traitement avec réactif de Lawesson, en dérivé éthanethioamide correspondant,
conversion dudit dérivé éthanethioamide en un dérivé (((diarylméthyle)sulfanyle)méthyle) thiazole, en particulier en 2-(diphényle méthane sulfanyle méthyle)-1,3-thiazole, 4-(diphényle méthane sulfanyle méthyle)- 1,3-thiazole, 2-(diphényle méthane sulfanyle méthyle)-4-méthyle- 1,3 -thiazole ou 4-(diphényle méthane sulfanyle méthyle)-2-méthyle-1,3-thiazole, et oxydation dudit composé thiazole en dérivé (diaryle méthane sulfinyle méthyle) thiazole correspondant.
